# EUROPEAN PATENT SPECIFICATION

(11) **EP 4 599 884 B1**
(45) Date of publication and mention of the grant of the patent: **11.11.2026**
(21) Application number: 25156323.5
(22) Date of filing: 06.02.2025
(51) Int. Cl.: A61N 1/39

(54) **SYSTEMS AND METHODS FOR CONFIGURABLE DEFIBRILLATOR DISPLAYS AND PATIENT CARE REPORTS**
SYSTEME UND VERFAHREN FÜR KONFIGURIERBARE DEFIBRILLATORANZEIGEN UND PATIENTENPFLEGEBERICHTE
SYSTÈMES ET PROCÉDÉS POUR AFFICHAGES DÉFIBRILLATEURS CONFIGURABLES ET RAPPORTS DE SOINS AUX PATIENTS

(30) Priority: 08.02.2024 US 202463551414 P
(43) Date of publication of application: 13.08.2025
(60) Divisional application: 26210050.6
(73) Proprietor: Physio-Control, Inc., Redmond, WA 98052 (US)
(72) Inventor: RUTZER, Mark, Redmond, 98052 (US); LIU, Michelle, Redmond, 98052 (US); DAYNES, John, Redmond, 98052 (US); STEARNS, Holly, Redmond, 98052 (US)
(74) Representative: V.O.

(56) References cited:
- US-A1- 2020 121 199
- US-A1- 2022 139 545
- US-A1- 2022 193 434

## Description

### BACKGROUND

Unless otherwise indicated herein, the description in this section is not prior art to the claims in this application and is not admitted to be prior art by inclusion in this section.

External defibrillators are designed to treat sudden cardiac arrest by delivering a controlled electric shock to the heart. There are several types of external defibrillators, including manual defibrillators, which are generally used by trained medical personnel and first responders, and automated external defibrillators, commonly known by the acronym AED, which are designed for use by laypersons. An AED is typically a small, portable device that analyzes the heart's rhythm and if the analysis determines that a defibrillating shock is advisable, either prompts the user to deliver a defibrillation shock or delivers a defibrillation shock without user interaction. Once a typical AED is activated, it can guide the user through each step of the defibrillation process by providing instructions in the form of aural or visual prompts.

The screen of an external defibrillator typically features a user-friendly graphical user interface ("GUI") with clear visual and/or audible prompts to guide individuals through the defibrillation process. For AEDs, designed to be used by laypersons, the GUI commonly shows physiological information, such as a heart rhythm analysis results, prompts for CPR, and instructions for electrode placement. Additionally, the GUI may indicate when a shock is advised and guide users on proper timing and technique, contributing to the device's overall accessibility and usability in high-stress situations. For manual defibrillators, designed to be used by trained medical personnel and first responders, the GUI will typically convey much more detailed information, such as patient vital signs, ECG data, and other patient information.

Regardless of defibrillator type, however, the way information is shown on the GUI of the external defibrillator is stationary. In other words, the GUI is in a pre-set format and cannot be configured by the user, regardless of the sophistication of the user. As background, an example of an external defibrillator for review of clinical data is described in US2020/121199.

### SUMMARY

The invention is defined by the independent claims. Embodiments of the invention are defined in the dependent claims. The present disclosure generally relates to a system comprising a processor that displays patient data on a GUI of an external defibrillator based on user preference and is designed to maximize the available space on the GUI. For example, a user can reconfigure the GUI to move waveform channels to a specific position based on the user's preference. Further, based on a determination of what data is available from patient sensors, the amount of data that the user wants to view and the relative importance of that data, the processor can change characteristics of the data to maximize the data shown on the GUI and/or the location where that data is shown on the GUI.

In one aspect, the present application describes a method. The method may include displaying, on a GUI of an external defibrillator, a first vital sign channel. The first vital sign channel can include a first input of real-time sensor data gathered by at least one sensor, and can be in a first position on the GUI. The method can further include detecting that a second sensor is connected to the external defibrillator. The second sensor gathers a second input of real-time sensor data. The method can also include determining a priority of the second real-time sensor data in comparison to the first real-time sensor data. Based on the priority, the method can include establishing a position for the second real-time sensor data on the GUI. The method can also include displaying, on the GUI, a second vital sign channel that includes the second input of real-time sensor data. The second vital sign channel can be in the established position on the GUI, and can be configured to change positions on the GUI. The method can additionally include detecting a user input. The user input can be a selection of either the first vital sign channel or the second vital sign channel. The method can further include changing at least one characteristic of the selected vital sign channel on the GUI. The at least one characteristic can be a position of the selected vital sign channel, a width of the selected vital sign channel, and a designation of the selected vital sign channel. Finally, the method can include displaying, on the GUI of the external defibrillator, the first vital sign channel and the second vital sign channel with the changed characteristic of the selected vital sign channel.

In another aspect, the present application describes an external defibrillator. The external defibrillator can include at least one sensor removably coupled to the external defibrillator, and a GUI configured to display a plurality of patient data. The GUI can include a first vital sign channel, a second vital sign channel, a third vital sign channel, and a menu. The first vital sign channel can include a first input of real-time sensor data gathered by the at least one sensor. The first vital sign channel can be in a first position on the GUI. The second vital sign channel can include a second input of real-time sensor data gathered by the at least one sensor. The second vital sign channel can be in a second position on the GUI, and configured to change positions. The third vital sign channel can include a third input of real-time sensor data gathered by the at least one sensor. The third vital sign channel can be in a third position on the GUI, and configured to change positions. The menu on the GUI can include a plurality of selectable options including a patient treatment event list. The patient treatment event list can include at least one reference to an event in the first real-time sensor data.

In yet another aspect, the present application describes a non-transitory computer-readable medium having computer-executable instructions stored thereon that, if executed by a computing system, cause the computing system to perform operations. The operations can include displaying, on a GUI of an external defibrillator, a first vital sign channel. The first vital sign channel can include a first real-time waveform based on data gathered by a first sensor. The operations can also include displaying, on the GUI of the external defibrillator, a second vital sign channel. The second vital sign channel can include a second real-time waveform based on data gathered by a second sensor. The operations can further include detecting a user input. The user input can be a selection of either the first vital sign channel or the second vital sign channel. The operations can additionally include changing at least one characteristic of the selected vital sign channel. The at least one characteristic can include a position of the selected vital sign channel, a width of the selected vital sign channel, and a primary designation of the selected vital sign channel. The operations can finally include displaying, on the GUI of the external defibrillator, the first vital sign channel and the second vital sign channel with the changed characteristic of the selected vital sign channel.

Further, the present application includes enabling a processor or user to add, delete or change positions of the sensor data displayed on a GUI that is independent of a defibrillator, but obtains sensor data that is being, has been or could be displayed on a defibrillator GUI in the same manner as described above. This would include, for example, at a tablet, laptop or desktop computer having a GUI that is displaying such sensor data and includes both real-time and post event display.

These as well as other aspects, advantages, and alternatives will become apparent to those of ordinary skill in the art by reading the following detailed description, with reference, where appropriate, to the accompanying drawings.

### BRIEF DESCRIPTION OF THE DRAWINGS

FIG. 1 is a diagram illustrating a representation of an example scene showing the use of an external defibrillator for monitoring and delivering treatment to a person or patient experiencing a medical condition.
FIG. 2 illustrates a front view of an external defibrillator.
FIG. 3 illustrates a simplified block diagram of the components of an external defibrillator.
FIG. 4 illustrates a GUI of an external defibrillator.
FIG. 5 illustrates a GUI of an external defibrillator.
FIG. 6A illustrates a GUI of an external defibrillator displaying real-time sensor data based on priority.
FIG. 6B illustrates a GUI of an external defibrillator displaying real-time sensor data based on priority.
FIG. 7 illustrates a GUI of an external defibrillator displaying a menu.
FIG. 8A illustrates a GUI of an external defibrillator displaying a patient treatment event list.
FIG. 8B illustrates a GUI of an external defibrillator displaying waveforms for a patient treatment event list.
FIG. 9 is a flowchart illustrating a method of displaying vital sign channels based on priority and preference.
FIG. 10 illustrates a GUI of an external defibrillator displaying vital sign channels.
FIG. 11 illustrates a GUI of an external defibrillator displaying vital sign channels.
FIG. 12 illustrates a GUI of an external defibrillator displaying vital sign channels.
FIG. 13A illustrates a GUI of an external defibrillator displaying real-time sensor data based on priority.
FIG. 13B illustrates a GUI of an external defibrillator displaying real-time sensor data based on priority.
FIG. 14 illustrates a GUI of an external defibrillator displaying vital sign channels.
FIGS. 15A, 15B and 15C illustrate a GUI of an external defibrillator displaying a vital sign channel removal.
FIG. 16 illustrates a GUI of an external defibrillator displaying a quick events menu.
FIG. 17 illustrates a GUI of an external defibrillator displaying a collapsed therapy overlay.
FIG. 18 illustrates a GUI of an external defibrillator displaying a non-collapsed therapy overlay.

### DETAILED DESCRIPTION

The figures and the following description illustrate specific example methods, systems, and/or non-transitory computer readable mediums. It will be appreciated that those skilled in the art will be able to devise various arrangements that, although not explicitly described or shown herein, embody the principles described herein and are included within the scope of the claims that follow this description. Furthermore, any examples described herein are intended to aid in understanding the principles of the disclosure and are to be construed as being without limitation. As a result, this disclosure is not limited to the examples described below, but by the claims and their equivalents.

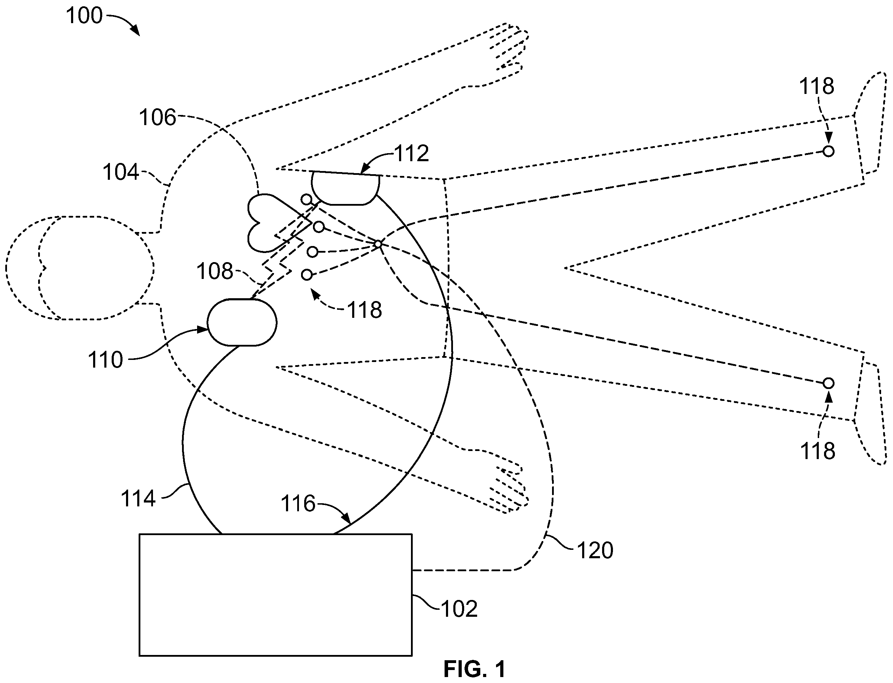

Particular example methods, systems, and/or non-transitory computer readable mediums are described herein with reference to the drawings. In the description, common features may be designated by common reference numbers throughout the drawings. In some drawings, multiple instances of a particular type of feature may be used. Although these features are physically and/or logically distinct, the same reference number is used for each, and the different instances are distinguished by addition of a letter to the reference number. When the features as a group or a type are referred to herein (e.g., when no particular one of the features is being referenced), the reference number is used without a distinguishing letter. However, when one particular feature of multiple features of the same type is referred to herein, the reference number is used with the distinguishing letter. For example, referring to Figure 1, sensors are illustrated and associated with reference number 118. When referring to a particular one of the sensors, such as the sensor 118A, the distinguishing letter "A" may be used. However, when referring to any arbitrary one of the sensor or to the sensors as a group, the reference number 118 may be used without a distinguishing letter.

As used herein, various terminology is used for the purpose of describing particular implementations only and is not intended to be limiting. For example, the singular forms "a," "an," and "the" are intended to include the plural forms as well, unless the context clearly indicates otherwise. Further, the terms "comprise," "comprises," and "comprising" are used interchangeably with "include," "includes," or "including." Additionally, the term "wherein" is used interchangeably with the term "where." As used herein, "exemplary" indicates an example, an implementation, and/or an aspect, and should not be construed as limiting or as indicating a preference or a preferred implementation. As used herein, an ordinal term (e.g., "first," "second," "third," etc.) used to modify an element, such as a structure, a component, an operation, etc., does not by itself indicate any priority or order of the element with respect to another element, but rather merely distinguishes the element from another element having a same name (but for use of the ordinal term). As used herein, the term "set" refers to a grouping of one or more elements, and the term "plurality" refers to multiple elements.

### I. Overview

A cardiac arrest is a life-threatening medical condition in which a person's heart fails to provide enough blood flow to support life. During a cardiac arrest, the electrical activity may be disorganized (ventricular fibrillation), too rapid (ventricular tachycardia), absent (asystole), or organized at a normal or slow heart rate (pulseless electrical activity).

The probability of surviving a cardiac arrest depends on the speed with which appropriate medical care is provided to a patient experiencing the cardiac arrest. External defibrillators can be used quickly to save lives. An external defibrillator is a small, portable device that analyzes the heart's rhythm and prompts a user to deliver treatment to the patient. Once the external defibrillator is activated, it can guide the first responder through each step of the treatment by providing audible and/or visual prompts, that may include CPR treatment and/or a defibrillation pulse if it determines the desirability for such a pulse. An external defibrillator can provide adequate instructions to the users to improve the overall success rate of treating cardiac arrest patients.

There are generally two types of external defibrillators, manual external defibrillators and automated external defibrillators, or "AEDs." Manual external defibrillators are generally designed for use by a first responder, who can be an emergency medical services worker, a firefighter, or a police officer, trained in using the device. An AED is an external defibrillator with automated functions and user-friendly guidance that allow a layperson with minimal or no training to operate the defibrillator. In many cases, manual external defibrillators have an automatic, or AED, mode as well. Using one of these types of external defibrillators, the first responder or layperson can deliver resuscitative therapies to the cardiac arrest patient.

Manual external defibrillators, however, which are intended to be operated by a trained first responders, typically have GUIs that are capable of displaying detailed patient data, including a range of vital signs, including ECG (electrocardiogram) waveforms, arterial oxygen saturation/pulse oximetry (SpO2) and end-tidal carbon dioxide (ETCO2) waveforms, pulse, temperature, heart rate, spO2 pulse rate, spO2 Oxygen saturation, carboxyhemoglobin concentration, methemoglobin concentration, respiration rate, carbon dioxide (CO2), invasive blood pressures, and non-invasive blood pressure, among other data on a screen.

The manual external defibrillator can use sensors to collect patient data and display the patient data for a user to review. The GUI can include waveforms of real-time data gathered as well as post-event displays of a waveform from start to finish based on the data gathered from the patient. Often times, there are multiple sensors connected to the manual external defibrillator gathering data from the patient. The data gathered by the sensors is displayed on the manual external defibrillator so that the first responder can monitor the patient's vitals and make healthcare decisions based on the vitals.

Currently, manual external defibrillators can display multiple waveforms as well as other patient vital signs data displayed as numbers. Typically, on the GUI of manual external defibrillators, the waveforms and other patient vital signs data are static, meaning they are displayed in a pre-determined configuration. For example, the waveforms may be displayed in a pre-programmed template in lines from top to bottom on the GUI with the other patient vital signs data at the bottom. Each waveform may span across the width of the screen.

To the extent that the manual external defibrillator may allow switching between different screens to provide different patient data, the data is typically displayed in predetermined templates. This can result in inefficient display of data on the limited screen real estate available on a manual external defibrillator, and prevent optimization of the GUI based on available data . For example, in the manual external defibrillator displays, there may be a limit on the number of waveforms and other vital signs that can fit. The screen may only be big enough to display five waveforms without compromising the quality of the visualization for the healthcare provider to review. Even if a waveform is removed, an empty space may be left instead of being filled by another waveform to utilize space.

In current manual external defibrillators, as previously mentioned, the GUI may also include a post-event waveform view. The post-event waveform view may include a plurality of waveforms based on data gathered from a patient from a start time to an end time. For example, the post-event waveform view could be compiled post-event in order to demonstrate all of the patient data gathered during an event. The post-event waveform view could also include bookmarks of specific sub-events that occurred during the event. The specific sub-events may be automatically added by the manual external defibrillator based on the data gathered.

The present disclosure generally relates to displaying patient data on an external defibrillator based on user preference. For example, data gathered from sensors can be displayed on the external defibrillator. When a sensor is connected to the external defibrillator, and the data gathered can be displayed in a channel as a waveform or other vital sign with appropriate labels indicating the type of data being displayed. If there are multiple channels to display, the processor of the external defibrillator may include logic to display the channels in the most efficient way possible, so that the maximum number of channels are displayed in an orientation that makes them easy to view and understand. Additionally, the first responder may be able to use touch to change channel widths and rearrange the channels on the GUI into orientations that suit the first responder's preference. Because the position of the channels can be changed, they can be considered dynamic. The first responder may also be able to store their preferred orientations and positions, so that the external defibrillator automatically displays the channels that way.

In an example implementation, the channel that displays an ECG waveform may be at the top of the GUI. This is because the ECG waveform may be considered the most relevant for the first responder. The channel may therefore be "pinned" to the top position. The pinned channel may be the only channel the first responder is not able to move. Even if there is no ECG waveform to display, the top vital sign channel may be reserved for ECG data, and may therefore remain blank. Alternatively, the labels for the ECG waveform may be displayed in the top vital sign channel, but there may be blanks or underscoring in the data area indicating no waveform data is being received. However, when there are multiple channels displaying ECG waveforms, the first responder may be able to designate the channel that they want to pin to the top of the display. The first responder can also change which channel is designated to be pinned to the top position.

When an additional sensor is connected to the external defibrillator, the aforementioned logic might be used to determine where to display the channel associated with the additional sensor on the GUI based on the priority of the data in all of the channels. For example, instead of adding the new channel below all of the channels already being displayed, the logic may be used to determine what position the new channel should be placed in based on data priority, and in order to do so, the remaining channels may be rearranged. The channels that are given a higher priority may be positioned closer to the top of the GUI. Even after this, the first responder can still use touch to rearrange the channels into their preferred orientations.

When one of the sensors is disconnected from the external defibrillator, the processor may be programmed to prevent the channel corresponding to the data gathered from the sensor from automatically disappearing from the GUI. This programming is designed to prevent the accidental removal of channels. In such a situation, in order to remove the channel after the sensor has been disconnected, the processor may be programmed to require the first responder to first touch the channel on the GUI to select it, select an option to remove the channel, and then select a confirmation to remove the channel. Once the confirmation is selected, the channel will disappear from the GUI. The remaining channels may fill in the position that the removed channel was in. Alternatively, the remaining channels can be re-prioritized to determine their position on the GUI.

The GUI of the external defibrillator can also display a patient treatment event list. The patient treatment event list can be accessed via a menu option. The patient treatment event list can display a list of events that occurred during patient treatment. The events may be automatically added to the list by the external defibrillator and can also be manually entered by the first responder. Each event can be linked to a waveform. For example, the event may be marked in the waveform at the time the event occurred during patient treatment. The events can be a shock, a pacing set, pacing stopped, pacing started, pacing changed, pacing paused, an alarm violation, print, 12/15 lead, administration of a shock, or administration of a medication. By selecting the event in the patient treatment event list, the waveform and time in the waveform corresponding to the event may be displayed GUI.

The first responder may be able to filter and rearrange all of the events in the patient treatment event list. For example, the events can be sorted in ascending or descending order based on the time the event occurred. The events could also be filtered based on whether the event is a treatment, medication, generic event, or 12/15 lead.

### II. Example Architecture

Referring now to the figures, Figure 1 is a diagram of a representation of an exemplary scene 100 showing use of an external defibrillator 102 for monitoring and providing treatment or therapy to a person 104 experiencing a medical condition, such as a cardiac arrest. The external defibrillator 102 may be operated by a user (e.g., a healthcare professional, service worker, a doctor, a first responder, etc.) and may be used in a hospital or a pre-hospital environment or setting. The external defibrillator 102 may include functions and operations of a manual defibrillator, an automatic external defibrillator (AED), or any other suitable defibrillator. In some examples, the external defibrillator 102 may be a monitor defibrillator, which is a combination of a monitor and a defibrillator.

As shown in Figure 1, the external defibrillator 102 is positioned near the person 104 (e.g., patient). The person 104 may be experiencing a condition in his or her heart 106, which could be, for example, cardiac arrest or any other cardiac rhythm abnormality. The person 104 may be lying on his or her back on a surface, such as the ground or a bed, and may be located in a hospital, a home, or a pre-hospital environment (e.g., an ambulance). The external defibrillator 102 may be configured to generate an electrical pulse 108 and deliver the electrical pulse 108 to the person 104. The electrical pulse 108, also known as a defibrillation shock or therapy shock, is intended to go through the chest of person 104 and restart the heart 106, in an effort to save the life of person 104. The electrical pulse 108 can further include one or more pacing pulses and the like.

The electrical pulse 108 may be delivered to the person 104 using defibrillation electrodes 110 and 112. The defibrillation electrodes 110 and 112 may include hand-held electrode paddles or electrode pads placed on the body of the person 104. An electrical cable 114 may connect the defibrillation electrode 110 to the external defibrillator 102 and an electrical cable 116 may connection the defibrillation electrode 112 to the external defibrillator 102. When the defibrillation electrodes 110 and 112 are in electrical contact with the body of person 104, the external defibrillator 102 may administer, via the defibrillation electrodes 110 and 112, the electric pulse 108 through the heart 106 of person 104. The defibrillation electrodes 110 and 112 may also be configured to sense or detect one or more physiological parameters of the person 104 and to generate signals representative of the physiological parameters.

As shown in phantom in FIG. 1, one or more sensors 118 may be placed at various locations on the body of the person 104. In an example implementation, at least some of sensors 118 can be electrodes. The sensors 118 may be configured to sense or detect physiological parameters of the person 104 and to produce signals representative of the physiological parameters. The sensors 118 can be removably coupled to the external defibrillator 102. In an example implementation, the sensors 118 could be ECG sensors. The sensors 118 could also include impedance sensors, temperature sensors, O2 sensors, blood pressure sensors, heart rate sensors, and CO2 sensors.

An electrical cable 120 may connect the sensors 118 to the external defibrillator 102. Alternatively, the sensors 118 could be in wireless communication with the external defibrillator 102. The physiological parameters generated by the sensors 118 and/or the defibrillation electrodes 110 and 112 may be provided to the external defibrillator 102 for analysis. The physiological parameters may include ECG data, heart rhythm data, heart rate data, cardiac output data, blood flow data, a level of perfusion, respiration data, body temperature data, and/or any other suitable physiological parameter that may be used to assess the physical condition of the person 104.

The external defibrillator 102 may be configured to select an appropriate treatment protocol based on the physiological parameters. For example, the external defibrillator 102 may determine a cardiopulmonary resuscitation (CPR) treatment protocol to apply to the person 104. The external defibrillator 102 may also determine whether a defibrillation pulse should be applied or delivered to the person 104. Further, the external defibrillator 102 may display the representations of the physiological parameters of the person to assist a user in treating and diagnosing medical conditions. In an example implementation, the physiological parameters can include ECG data, invasive blood pressure, CO2, SpO2, non-invasive blood pressure, and temperature. The physiological parameters may be displayed in the order of ECG waveforms at the top, followed by invasive blood pressure, followed by EtCO2, followed by non-invasive blood pressure, followed by temperature.

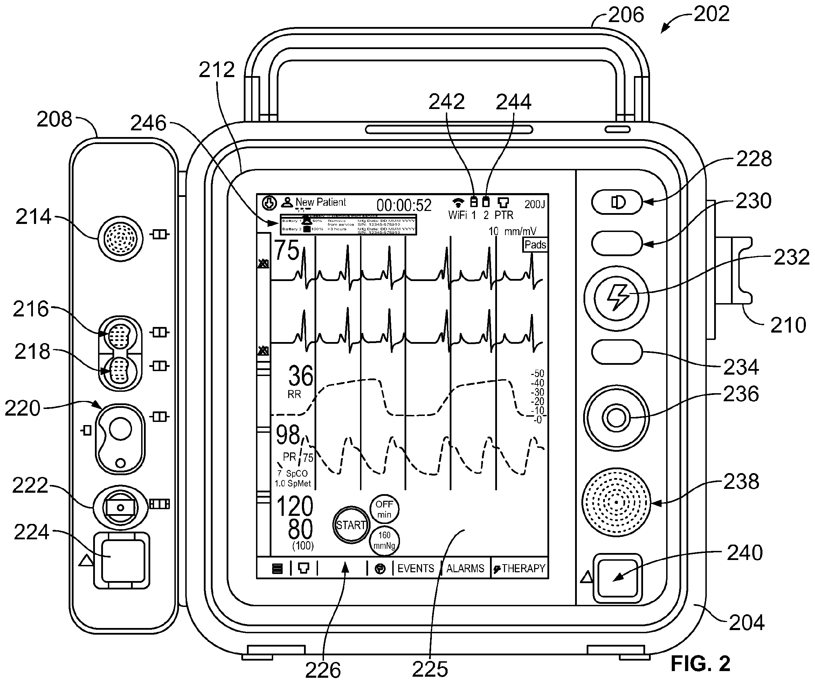

Figure 2 illustrates a front view of an external defibrillator 202, in accordance with an example implementation. The external defibrillator 202 can comprise the external defibrillator 102 of Figure 1. The external defibrillator 202 may be configured to monitor and provide treatment or care to a person or patient experiencing a medical condition, such as a cardiac arrest or any other cardiac rhythm abnormality. The external defibrillator 202 may be operated by a user (e.g., a medical professional, a first responder, a healthcare professional, service worker, a doctor, etc.) and may be used in a hospital or a pre-hospital environment or setting. As illustrated, the external defibrillator 202 may be a monitor defibrillator, which is a combination of a monitor and a defibrillator.

As a defibrillator, the external defibrillator 202 may be configured to deliver an electrical pulse or shock to a person experiencing a medical condition. The external defibrillator 202 may be configured to operate or function in one or more defibrillation modes, such as a manual defibrillation mode, an AED mode, or any other suitable defibrillation mode. For example, the external defibrillator 202 may be selected to operate in an AED mode when the external defibrillator 202 is intended to be used by first responders and/or people who are not trained in providing medical treatment using defibrillation. When operating in the AED mode, the external defibrillator 202 can determine whether a defibrillation pulse or shock is needed and, if so, charge an energy storage device (e.g., a capacitor) of the external defibrillator 202 to a predetermined energy level and instruct the user to administer the defibrillation shock. The external defibrillator 202 may also be selected to operate in a manual defibrillation mode when the external defibrillator 2020 is intended to be used by persons who are trained to provide medical treatment using defibrillation (e.g., medical professionals, such as doctors, nurses, paramedics, emergency medical technicians, etc.). When the external defibrillator 202 is configured to operate in the manual defibrillation mode, the device provides an array of patient vital signs to the user, including the patient's ECG data (as discussed below in connection with Fig. 2), allowing user interpretation of the patient vital signs, and allows the user to change the selected energy (although configuration options may set a default energy), charge the defibrillator energy storage capacitor, and deliver a shock to the patient.

As a monitor, the external defibrillator 202 can monitor and evaluate physiological parameters of a person being treated for a medical condition. The external defibrillator 202 may receive or acquire signals or voltage from one or more electrodes or sensors placed at various locations on the body of the person. The electrodes may sense or detect the physiological parameters of the person and produce signals representative of the physiological parameters. The representations of the physiological parameters may be displayed by the external defibrillator 202 to assist a user in treating and diagnosing medical conditions of a person. The physiological parameters may include ECG data, body temperature, non-invasive blood pressure (NIBP), SpO2 data, a concentration or partial pressure of carbon dioxide in the respiratory gases (e.g., capnography), and/or any other suitable physiological parameter of a person. These physiological parameters of the person can be stored in the memory of the external defibrillator 202. In some examples, the physiological parameters may be transmitted to other remote communication or computing devices and databases.

As shown in Figure 2, the external defibrillator 202 includes a housing or casing 204, a handle 206, an input module or interface 208, a defibrillation interface 210, and a user interface 212. The handle 206 of the external defibrillator 202 is attached to the housing 204 to allow a user to move or transport the external defibrillator 202 to a location near a person experiencing a medical condition. The housing 204 may be generally rectangular or square shaped. In other examples, the housing 204 can have any other suitable shapes. The housing 204 may be formed from various materials or combinations of materials. For example, the housing 204 may be made of molded plastic, metal, or some combination of both.

The input module 208 of the external defibrillator 202 may be coupled to or integral with the housing 204. The input module 208 may enable the external defibrillator 202 to receive vital signs and other physiological parameters (e.g., a heart rate (HR) and ECG data) of a person via sensors (e.g., the sensors 118 of Figure 1). The sensors may be placed on the body of a person to sense or detect signals generated by the body or heart of the person. The input module 208 may include one or more ports or receptacles to enable the sensors to be detachably coupled to the input module 208. As shown in Figure 2, the input module 208 can include a port 214 configured to be connected to an oxygen saturation (SpO2) sensor, a port 216 configured to be connected to a temperature sensor, a port 218 configured to be connected to a sensor for measuring invasive blood pressure (IP) via a catheter, a port 220 configured to be connected to a sensor for measuring partial pressure of carbon dioxide (CO2) in gases in the airway via capnography, and a port 222 configured to be connected to sensor for measuring a non-invasive blood pressure (NIBP). The input module 208 may also include a communication port 224, such as a Universal Serial Bus (USB) port, that can be used to connect to an input device (e.g., a mouse, a keyboard, etc.). The input module 208 may include other ports to enable any other suitable physiologic parameter of a person to be monitored and evaluated by the external defibrillator 202.

The defibrillation interface 210 of the external defibrillator 202 is configured to enable electrical charges or pulses to be delivered or applied to a person via defibrillation electrodes (e.g., the defibrillation electrodes 110 and 112 of Figure 1). The defibrillation electrodes may also be configured to enable the external defibrillator 202 to receive physiological parameters of a person (e.g., a heart rate (HR), ECG data, etc.). The defibrillation interface 210 may include one or more ports or receptacles capable of allowing defibrillation electrodes to be detachably coupled to the defibrillation interface 210. A cable assembly or therapy cable may enable the defibrillation electrodes to be coupled to the ports of the defibrillation interface 210. Each therapy cable may include a defibrillation electrode attached at one end and a connector attached to the other end. The connector may be configured to be coupled to or plugged into a port or receptacle of the defibrillation interface 210.

The user interface 212 of the external defibrillator 202 may include one or more input devices configured to receive inputs or commands from a user and one or more output devices configured to provide information to the user. The input devices of the user interface may include various controls, such as electronic or mechanical switches, pushbuttons, keyboards, touchscreens, microphones, scanners, and/or cameras, etc., for operating the external defibrillator 202. The output devices of the user interface 212 can be visual, audible or tactile, for communicating to a user of the external defibrillator 202 (e.g., a medical professional, a first responder, etc.). For example, the output devices may be configured to present visual alarms or alerts, flashing lights, and/or warnings to the user. The output devices may also include an audio system that provides audio signals to aurally communicate with the user voice prompts that deliver instructions or commands, monotonal, ascending, descending or quickening tones to indicate alerts or warnings, or any other suitable audio signals for communicating with the user of the external defibrillator 202.

As shown in Figure 2, the user interface 212 of the external defibrillator 202 includes a power button 228 configured to turn the external defibrillator on and off (e.g., "On-Off" button), a charge button 230 configured to cause the external defibrillator 202 to build an electric charge to be applied to the person in a form of a defibrillation shock or pulse, a defibrillation shock button 232 configured to cause the external defibrillator 202 to apply a defibrillation pulse or therapy shock to a person during a defibrillation episode, an analyze button 234 configured to cause the external defibrillator 202 to analyze physiologic parameters of a person (e.g., ECG data) to facilitate determining the appropriate time to apply a defibrillation pulse or shock, and a speed dial button 236 configured to navigate through menus of on-screen software. The user interface 212 may also include a speaker 238 to provide audio output and a USB output port 240 to facilitate connecting the external defibrillator 202 to a device such as a printer. The user interface 212 may include any other suitable output device for providing outputs or input device for receiving inputs from a user.

As shown in Figure 2, the user interface 212 of the external defibrillator 202 may also include a display device 225 (e.g., a touchscreen) for displaying a GUI 226. The GUI 226 can display physiologic parameters of a person (e.g., a patient), provide visual feedback to the user (e.g., a healthcare provider) about a condition of the person, provide information about the external defibrillator 202, and allow the user to interact with and operate the external defibrillator 202. The GUI 226 may also be configured to visually present various measured or calculated parameters associated with the person indicating the physical status of the person (e.g., an ECG), and/or instructions and/or commands, including prompts to perform cardiopulmonary resuscitation (CPR) treatment or other treatment instructions, to the user. Further, the GUI 226 may include multiple visual user interface items that are selectable or "clickable" by the user including user-selectable icons, user-selectable on-screen buttons, menus, widgets, scroll bars, graphical objects, and other items for facilitating user interaction.

As shown in Fig 2, the GUI 226 includes graphical representations of a first battery unit indicator 242 and a second battery unit indicator 244. The first battery unit indicator 242 and second battery unit indicator 244 are configured to display a status of a first battery unit and a status of the second battery unit, respectively. For example, the first and second battery unit indicators 242 and 244 may provide a charging level of the first and second battery units. The GUI 226 may also display messages and information about the end of life of a first battery unit and/or a second battery unit. As shown, the GUI 226 may indicate that the first battery unit should be removed from service. The GUI 226 may also provide additional information about the battery units of the external defibrillator 202, such as battery charge levels (e.g., a remaining charge), manufacturing dates, serial numbers, etc. Further, the GUI 226 may provide audible alarms or warnings when a battery unit of the external defibrillator 202 has reached its end of life and/or should be removed and replaced.

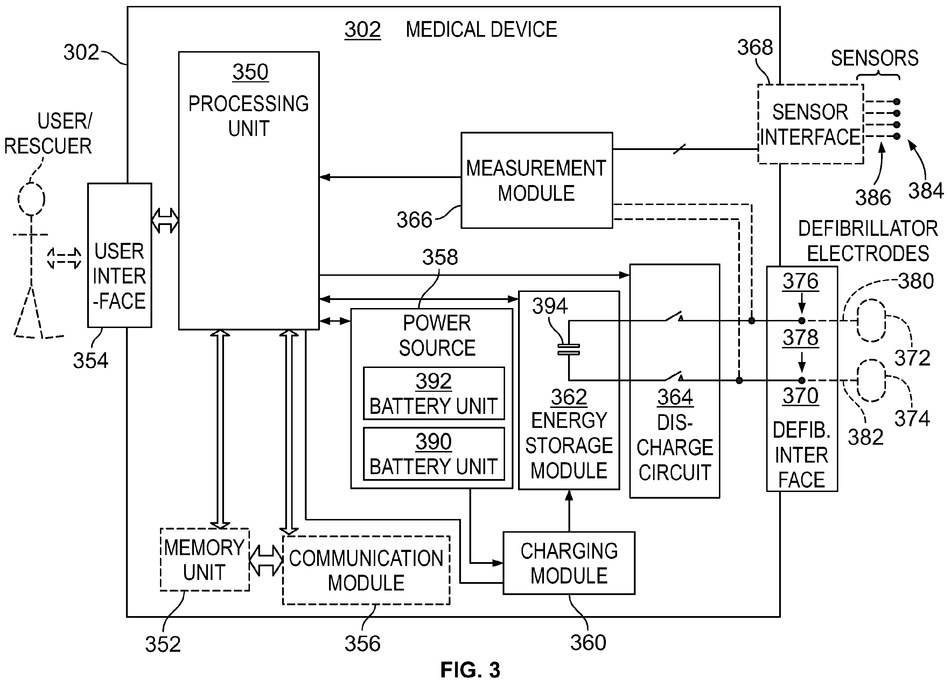

FIG. 3 is a diagram showing components of an external defibrillator 302. As shown in Figure 3, the external defibrillator 302 includes a processing unit 350, a memory unit 352, a user interface 354, a communication module or interface 356, a power source 358, a charging module 360, an energy storage module or device 362, a discharge circuit 364, a measurement module 366, a sensor interface 368, and a defibrillator interface 370. These components can be, for example, included in the external defibrillators of Figure 1 or 2.

The defibrillation interface 370 of the external defibrillator 302 may be configured to enable an electrical pulse or shock to be delivered or applied to a person (e.g., a patient) experiencing a medical condition. The defibrillation interface 370 may comprise the defibrillation interface 210 of Figure 2. The defibrillation interface 370 may include one or more ports or nodes 376 and 378 to enable the defibrillation electrodes 372 and 374 to be detachably coupled to the defibrillation interface 370. A cable assembly or therapy cable 380 may enable a defibrillation electrode 372 to be coupled to the port 376 of the defibrillation interface 370 and a cable assembly or therapy cable 382 may enable a defibrillation electrode 374 to be coupled to the port 378 of the defibrillation interface 370 of the external defibrillator 302. The connectors of the cable assemblies 380 and 382 may be configured to be coupled to or plugged into the ports 376 and 378 of the defibrillation interface 370. The connectors can be male or female connectors that are compatible with the ports 376 and 378 of the defibrillation interface 370 of the external defibrillator 302.

The defibrillation interface 370 may also enable the external defibrillator 302 to receive physiological parameters (e.g., a heart rate (HR) and ECG data) of the person from the defibrillation electrodes 372 and 374. Each of the defibrillation electrodes 372 and 374 may be configured to measure one or more physiological parameters of the person (e.g., heart electrical activity, heart rate, etc.) and to provide signals representative of the physiological parameters to the defibrillation interface 370. For example, when the defibrillation electrodes 372 and 374 are placed on the chest of the person, an ECG signal of the person can be detected as a voltage difference between the defibrillation electrodes 372 and 374. The defibrillation electrodes 372 and 374 may also be used to determine device parameters indicative of a condition of the external defibrillator, such as an electrode impedance or a user interaction with the external defibrillator 302. For example, the external defibrillator 302 can detect an impedance between the defibrillation electrodes 372 and 374 to determine whether the defibrillation electrodes 372 and 374 are making sufficient electrical contact with a person's body.

The sensor interface 368 of the external defibrillator 302 may also be configured to receive physiological parameters (e.g., a heart rate (HR) and ECG data) from one or more sensors 384. The sensors 384 may be placed in contact with the body of a person being monitored or treated for a medical condition. Each of the sensors 384 may include a transducer configured to sense a signal or voltage of the person. For example, the sensors 384 may measure or detect heart electrical activity, such as an electrocardiogram (ECG), saturation of the hemoglobin in arterial blood with oxygen (SpO2), carbon monoxide (carboxyhemoglobin, COHb) and/or methemoglobin (SpMet), partial pressure of carbon dioxide (CO2) in gases in the airway by means of capnography, total air pressure in the airway, flow rate or volume of air moving in and out of the airway, blood flow, blood pressure (e.g., non-invasive blood pressure (NIBP)), core body temperature with a temperature probe in the esophagus, oxygenation of hemoglobin within a volume of tissue (rSO2), and any other physiological parameters of a person being monitored or treated.

The sensor interface 368 may include one or more receptacles or ports (e.g., an ECG port) to enable the sensors 384 (e.g., physiological sensors) to be detachably coupled to the external defibrillator 302. The sensors 384 may be attached to the sensor interface 368 by cable assembles or therapy cables 386. In some implementations, the sensors 384 may be fixedly connected to the sensor interface 368. The therapy cables 386 may each include a sensor 384 at one end and to a connector at the opposite end. The connector can be configured to be coupled to or plugged into a port or receptacle of the sensor interface 368.

The measurement module 366 of the external defibrillator 302 may be configured to receive signals or sensor data from the sensor interface 368 and the defibrillation interface 370. The signals may be representative of physiological parameters of a person being monitored and/or treated for a medical condition. The measurement module 366 may measure or determine various physiological parameters from the signals. For example, the measurement module 366 may determine an ECG of the person based on a voltage difference between the defibrillation electrodes 372 and 374. The measurement module 366 may also determine device parameters indicative of a condition of the external defibrillator 302 such as an electrode impedance or a user interaction with the external defibrillator 302. For example, the measurement module 366 may measure an impedance or voltage across the defibrillation electrodes 372 and 374 or a pair of sensor.

The measurement module 366 may also include a filter circuit or hardware (e.g., amplifiers, filters, etc.) to attenuate and/or filter at least some of the noise that may be present on the signals received from the sensor interface 368 and/or the defibrillation interface 370. For example, the filter circuit may apply at least one filter to the signal to remove artifacts resulting from chest compressions being delivered to the person. The filter may be implemented as an analog filter, a digital filter, or combinations of both. The measurement module 366 may also digitize the signals received from the sensor interface 368 and/or defibrillation interface 370 prior to transmitting the signals to the processing unit 350.

The memory unit 352 of the external defibrillator 302 is in operable communication with the processing unit 350. The memory unit 352 may store various values, look-up tables, equations, audio and video files, and/or plurality of treatment protocols that can be read and accessed by the processing unit 350. The treatment protocols may include instructions regarding CPR treatment (e.g., chest compressions). The memory unit 352 can also store a person's sensed physiological parameters, historical data, lengths of time and rate of CPR treatments, and defibrillation pulses previously discharged. Further, the memory unit 352 can store instructions or computer executable code of software routines that can be retrieved and executed by the processing unit 350.

The memory unit 352 may include one or more computer-readable storage media that can be read or accessed by the processing unit 350. The computer-readable storage media can include volatile and/or non-volatile memory, dynamic random-access memory (DRAM) read-only memory (ROM), random access memory (RAM), magnetic disk storage media, optical storage media, smart cards, flash memory devices, or any other suitable memory. The computer-readable storage media can be integrated in whole or in part with the processing unit 350. The computer-readable storage media may be implemented using a single physical device (e.g., one optical, magnetic, organic or other memory or disc storage unit), while in other examples, the computer readable storage media can be implemented using two or more physical devices.

The processing unit 350 of the external defibrillator 302 is configured to control various operations of the external defibrillator 302. The processing unit 350 may receive inputs from various components of the external defibrillator 302 and process the inputs to produce outputs that may be stored in the memory unit 352 and/or displayed on the user interface 354. For example, the processing unit 350 may receive and evaluate physiological parameters of a person (e.g., electrical activity of the heart) from the sensors 384 and/or the defibrillation electrodes 372 and 374 placed on a person. The processing unit 350 may also determine whether a defibrillation pulse should be delivered to a person based upon the physiological parameters. For example, the processing unit 350 may make a shock/no shock determination based on ECG data or other information. In some examples, the processing unit 350 may cause the external defibrillator 302 to automatically deliver defibrillation pulses in AED mode or may advise the user of these determinations via the user interface 354. When a defibrillation shock has previously been delivered, the processing unit 350 may evaluate the efficacy of the delivered defibrillation pulse by determining if the heart of the person is still fibrillating based on the sensed electrical activity in order to determine whether an additional defibrillation pulse is warranted.

The processing unit 350 may also determine whether to commence charging of the energy storage module 362. This processing unit 350 may determine the rate to charge the energy storage module 362. Further, in manual mode, the processing unit may cause an electrocardiogram (ECG) to be displayed on the user interface 354 that reflects the sensed electrical activity of a heart of a person. In addition, the processing unit 350 may control delivery of other types of treatment therapy to the person via the defibrillation electrode 372 and 374, such as cardioversion or pacing therapy.

The processing unit 350 may include one or more general-purpose processors, special purpose processors (e.g., digital signal processors (DSP), application specific integrated circuits (ASIC), graphic processing units, etc.), or any other suitable processing unit or controller. The processing unit 350 can be configured to execute instructions (e.g., computer-readable program instructions) that are stored in memory and may be executable to provide the functionality of the external defibrillator described herein. For example, the processing unit 350 can execute instructions for causing the external defibrillator to display an ECG waveform on the user interface 354 of the external defibrillator 302.

The user interface 354 of the external defibrillator 302 facilitates user interactions with the external defibrillator 302. The user interface may comprise the GUI 226 of Figure 2. The user interface 354 may include various types of input devices for receiving inputs or commands from a user. For example, the input devices may include keyboards, electrical or mechanical switches, microphones, pushbuttons, touchscreens, scanners, and/or any other suitable input device for enabling inputs to the external defibrillator 302. For instance, a user can use interface items displayed on the GUI to input information regarding a particular event (e.g., a treatment or medication administered to the person). Additionally, the user interface can be used to rearrange the waveforms displayed on the external defibrillator 302, as described in further detail below.

The user interface 212 may also comprise various types of output devices for providing information to the user. The output devices of the user interface can be visual, audible or tactile for communicating to or providing feedback to a user (e.g., a rescuer, a first responder, a healthcare professional, etc.). The output devices may include a screen, one or more light emitting diodes (LEDs), and/or a speaker to output various sounds (e.g., voice or audio), etc. For example, the output device can also be configured to present visual alarms or alerts, flashing lights, and/or warnings to the user of the external defibrillator. The output device may also include an audio system 135 that provides an audio signal to aurally communicate with user voice prompts that deliver instructions or commands, monotonal, ascending, descending or quickening tones to indicate alerts or warnings, or any other suitable output device for communicating with the user.

The communication module 356 of the external defibrillator 302 may be in communication with the processing unit 350. The communication module 356 may enable patient data, treatment information, CPR performance, system data, environmental data, etc. to be communicated between the external defibrillator 302 and other devices, such as a remote assistance center and/or any other remote computing device. The communication module 356 may include one or more wireless or wireline interfaces that allow for both short-range communication and long-range communication to one or more networks or to one or more remote devices. The wireless interfaces may provide for communication under one or more wireless communication protocols, such as Bluetooth, Wi-Fi (e.g., an institute of electrical and electronic engineers (IEEE) 802.xx protocol), Long-Term Evolution (LTE), cellular communications, near-field communication (NFC), radio-frequency identification (RFID), and/or other wireless communication protocols. Such wireline interfaces may include an Ethernet interface, USB interface, or similar interface to communicate via a wire, a twisted pair of wires, a coaxial cable, an optical link, a fiber-optic link, or other physical connection to a wireline network.

The power source 358 of the external defibrillator 302 may provide power to the components of the external defibrillator 302. The power source 358 may include one or more battery units. The battery units may include lithium batteries, alkaline batteries, nickel cadmium batteries, nickel metal hydride batteries, or any other suitable type of battery or energy device.

The charging module 360 of the external defibrillator 302 may be configured to charge the energy storage module or device 362 before a defibrillation shock is delivered to a person. The charging module 360 may include charging circuitry, such as a flyback charger, for charging the energy storage module 362 to a selected voltage level that is determined based on a selected energy level for the defibrillation shock. During operation of the external defibrillator 302, the charging module 360 may draw power from the power source 358 to charge the energy storage module 362.

The energy storage module 362 of the external defibrillator 302 can store electrical energy in the form of an electrical charge, for delivery of a defibrillation pulse or shock to a person being treated for a medical condition. The energy storage module 362 can be charged by the charging module 360 to a desired energy level (e.g., between 50 Joules to 360), as controlled by processing unit 350. For instance, for an adult, the processing unit 350 can select an energy level from an adult energy sequence that includes energy levels of 200 Joules, 300 Joules, and 360 Joules. For a pediatric patient, the processing unit 350 can select an energy level from a pediatric energy sequence that includes energy levels of 50 Joules, 75 Joules, and 90 Joules. In some implementations, the energy storage module 362 may include one or more capacitors that stores the energy to be delivered to a person as a defibrillation shock. When the energy of the energy storage module 362 reaches the desired energy level, the defibrillation shock may be delivered manually or automatically. For example, the user interface 354 may provide an indication to the user that external defibrillator 302 is ready to deliver a defibrillation pulse or shock.

The discharge circuit 364 of the external defibrillator can enable the energy stored in the energy storage module 362 to be discharged to a person. The electrical energy may be discharged at a selected energy level, via the ports or nodes 376 and 378, to the defibrillation electrodes 304, 308 to cause a shock to be delivered to the person. The discharge circuit 364 can be controlled by the processing unit 350, or directly by the user via the user interface 354. In some implementations, the discharge circuit 355 can include one or more switches that, when activated, couple the energy storage module 362 to the ports or nodes 376 and 378 to deliver a defibrillation shock to person via defibrillation electrodes 372 and 374. For example, the processing unit 350 may activate the switches to electrically connect energy storage module 362 to the defibrillation electrodes 372 and 374, and thereby deliver the defibrillation shock to person. The switches can be made in a number of ways and may be formed, for example, of electrically operated relays. Alternatively, the switch may comprise an arrangement of solid-state devices such as silicon-controlled rectifiers or insulated gate bipolar transistors.

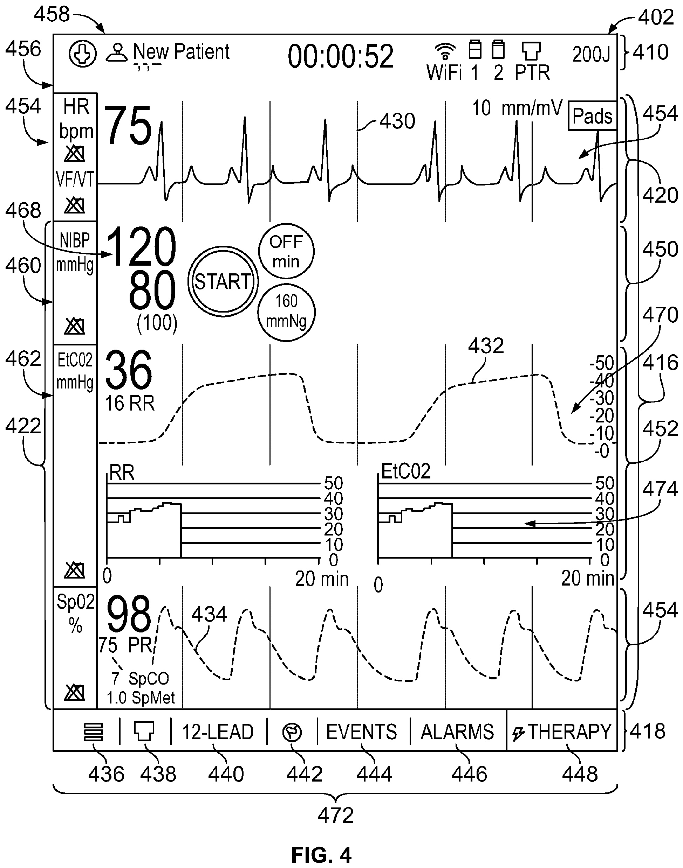

Figure 4 illustrates a GUI 402, in accordance with an example implementation. The GUI 402 can be displayed by an external defibrillator, such as the external defibrillator of Figures 1, 2, and 3. For example, an external defibrillator may be configured to generate a display of or visually present the GUI 402 on a screen, such as a touchscreen, to enable a user (e.g., a healthcare professional) to interact with the external defibrillator through user-selectable on-screen graphical items or components (e.g., buttons, menus, widgets, scroll bars, graphical objects, audio indicators, icons, etc.). The user-selectable user-interface items can convey information and represent actions that can be taken by a user. The user can select the user-selectable user-interface items by pressing or selecting areas on the touchscreen displaying the items. These user-selectable user-interface items can be enhanced with sounds, or visual effects like change in color, transparency, or drop shadows to facilitate interaction with the GUI 402.

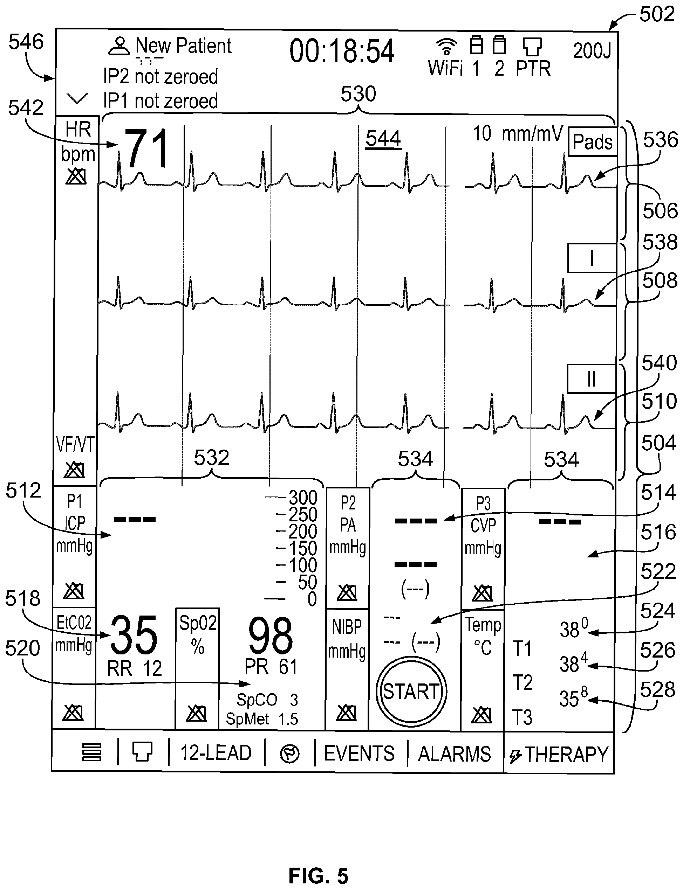

As shown in Figure 4, the GUI 402 includes a status bar 410, a messaging area 412, a content area 416, and a navigation task bar 418. The content area 416 of the GUI 402 may show patient data including physiologic parameters and waveforms output or displayed by the external defibrillator as well as provided by physiologic sensors. As shown in Fig. 4 and 5, the data is displayed along with appropriate labels (e.g., "HR bpm," "VF/VT," "SpO2 %," "EtCO2 mmHg," and "P1 ICP mmHg," for the respective physiological parameters and "Pads," "I" and "II," etc. for the ECG waveforms). The content area 416 can include multiple vital sign channels, including a first vital sign channel 420 for displaying a primary waveform 430 and multiple secondary channels 422 for displaying secondary data, such as a secondary waveform 432. In Figure 4, the primary waveform 430 is shown as an ECG waveform, obtained using defibrillation electrodes or sensors. In some examples, the secondary channels 422 may display one or more ECG waveforms as well as waveforms indicative of other physiological data (e.g., a plethysmograph, a capnography waveform, etc.). The secondary channel 422 may also display numerical values, such as a heart rate, respiration rate, a body temperature, and any other physiological parameters. As illustrated, the first vital sign channel 420 is positioned above the secondary channels 422. Although multiple secondary channels 422 are shown, in other examples, a GUI may only display a single channel.

The GUI 402 may also be configured to display waveforms next to a side rectangle having a particular color and labelled by a physiologic parameter to which the waveform pertains. For example, the GUI includes the waveform 430 for heart rate (HR), the waveform 432 for End-tidal CO2 (EtCO2), which indicates the partial pressure or maximal concentration of carbon dioxide (CO2) at the end of an exhaled breath, and the waveform 434 for SpO2. The GUI can also display NIBP values for a patient or person being monitored and/or treated. Further, the GUI 402 may display a navigation task bar 418 at the bottom of the GUI 402. The navigation task bar 418 may have various tabs and menu options. As shown, the navigation task bars 418 includes a menu button 436, a print button 438, a 12-Lead button 440, a generic event button 442, an events button 444, an alarms button 446, and therapy button 448.

In an example implementation, the GUI 402 of the external defibrillator can display vital sign channels in an order which the user can rearrange based on preference. The vital sign channels may be an input of sensor data gathered by the sensors coupled to the external defibrillator, as previously described. Multiple vital sign channels may be displayed on GUI 402 from top to bottom. The top vital sign channel may display an ECG waveform and may be pinned in place at a top position. The user may be able to change the positions of all of the subsequent vital sign channels. The GUI 402 of the external defibrillator can also display a menu with a patient treatment event list.

The external defibrillator can include at least one sensor removably coupled to the external defibrillator, as previously illustrated and described with reference to Figures 1, and 3. Additionally, as described in Figure 4, the external defibrillator can include the GUI 402. In an example implementation, GUI 402 further includes a first vital sign channel 420, a second vital sign channel 450, and a third vital sign channel 452. The first vital sign channel 420 may be in a first position 454 at a top 456 of a display 458 on the GUI 402. The second vital sign channel 450 may be in a second position 460 on the GUI 402 below the first vital sign channel 420. The second vital sign channel 450 may be configured to change positions on GUI 402. The third vital sign channel 452 may be in a third position 462 on the GUI 402. The third vital sign channel 452 may be configured to change positions on GUI 402. The first vital sign channel 420 can include a first input of real-time sensor data 464 gathered by the at least one sensor. The second vital sign channel 450 can include a second input of real-time sensor data 468 gathered by the at least one sensor. The third vital sign channel 452 can include a third input of real-time sensor data 470 gathered by the at least one sensor. GUI 402 can further include a menu, such as events menu button 444. The events menu 444 can include a plurality of selectable options including a patient treatment event list. The patient treatment event list can include at least one reference to an event in the first real-time sensor data.

In addition to the first vital sign channel 420, the second vital sign channel 450, and the third vital sign channel 452, GUI 402 can display up to thirteen vital sign channels and as few as one vital sign channel. Each vital sign channel displayed on GUI 402 may include a width 472. For example, each of the first vital sign channel 420, the second vital sign channel 450, and the third vital sign channel 452 have a width 472 that spans across the display 458 of GUI 402. As illustrated in Figure 4, the width 472 is full width. However, the width of other vital sign channels could also be reduced to half width and quarter width.

Figure 5 illustrates a GUI 502, such as the GUI 402 of Figure 4. The GUI 502 can be displayed by an external defibrillator, such as the external defibrillator of Figures 1, 2, and 3. The GUI 502 includes a plurality of vital sign channels 504, including a first vital sign channel 506, a second vital sign channel 508, a third vital sign channel 510, a fourth vital sign channel 512, a fifth vital sign channel 514, a sixth vital sign channel 516, a seventh vital sign channel 518, an eighth vital sign channel 520, a ninth vital sign channel 522, a tenth vital sign channel 524, an eleventh vital sign channel 526, and a twelfth vital sign channel 528. As illustrated, the first vital sign channel 506, the second vital sign channel 508, and the third vital sign channel 510 may be displayed on GUI 502 at full width 530. The fourth vital sign channel 512 may be displayed on GUI 502 at half width 532. The fifth vital sign channel 514, the sixth vital sign channel 516, the seventh vital sign channel 518, the eighth vital sign channel 520, and the ninth vital sign channel 522 may be displayed on GUI 502 at quarter width 534. The tenth vital sign channel 524, the eleventh vital sign channel 526, and the twelfth vital sign channel 528 may display temperature on GUI 502 at quarter width 534, and the three vital sign channels may be stacked on top of each other.

At full width 530, the vital sign channels, including the first vital sign channel 506, the second vital sign channel 508, and the third vital sign channel 510 can include a trend graph. For example, Figure 4 displays a full width of the third vital sign channel 452 which includes physiologic data about EtCO2, including EtCO2 trend graph 474. However, the vital sign channels at full width 530 do not need to display trend graphs.

Trends are hidden when the vital sign channel enters quarter width. At quarter width the vital sign channels, including the first vital sign channel 506, the second vital sign channel 508, and the third vital sign channel 510 do not include the trend graph. Figure 5 displays a quarter width 534 of the seventh vital sign channel 518, which includes physiologic data about EtCO2. Because it is at quarter width 534, no trend data is displayed.

The data displayed in the vital sign channels on the GUI 502 may be gathered by the sensors coupled to the external defibrillator, such as the sensors of Figures 1 and 3. For example, the first vital sign channel 506 can include a first input of real-time sensor data 536 gathered by the at least one sensor. The at least one sensor can collect real-time physiological data by monitoring the patient's cardiac activity. The ECG data provides information on the heart's rhythm and rate, crucial for identifying life-threatening arrhythmias. Additionally, impedance sensors measure the resistance to electrical flow in the chest. This combined data is analyzed by the external defibrillator's internal algorithms, which assess the patient's condition, determine the presence of shockable rhythms, and guide the device to deliver timely and appropriate electric shocks. The integration of sensor data enables the defibrillator to provide accurate feedback and instructions, enhancing its effectiveness in treating sudden cardiac arrest. The real-time data gathered by the sensors can then be displayed on GUI 502.

In one implementation, the second vital sign channel 508 can include a second input of real-time sensor data 538 gathered by the at least one sensor, and the third vital sign channel 510 can include a third input of real-time sensor data 540 gathered by the at least one sensor. For example, each of the vital sign channels could display ECG data gathered by the at least one sensor. Figure 5 illustrates an example in which the first vital sign channel 506, second vital sign channel 508, and third vital sign channel 510 all display ECG data gathered by the at least one sensor. Each vital sign channel could display an ECG waveform from a different sensor lead. Alternatively, the vital sign channels could include inputs of real-time sensor data gathered by other sensors. For example, at illustrated in Figure 5, the fourth vital sign channel 512, fifth vital sign channel 514, and sixth vital sign channel 516 display invasive blood pressure gathered by a sensor for measuring IP via a catheter.

In an example, as illustrated in Figure 5, the first input of real-time sensor data 536 in the first vital sign channel 506 is an ECG waveform 542. Since an ECG waveform is a necessary parameter, at least one ECG waveform should be displayed on GUI 502 at all times. Additionally, vital sign channels displaying an ECG waveform may be full width 530 at all times. In an example where the first vital sign channel 506 is the only one vital sign channel with an ECG waveform displayed on GUI 502, the processor may, for example, preclude a user from moving the ECG waveform from the top position on the GUI, or removed from being displayed on GUI 502 altogether.

Aside from an ECG waveform, the first input of real-time sensor data 536 in the first vital sign channel 506 could alternatively be an EtCO2 waveform, an SpO2 waveform, temperature, heart rate, carboxyhemoglobin concentration, methemogobin concentration, respiration rate, CO2, invasive blood pressure, and non-invasive blood pressure. Similarly, the second input of real-time sensor data 538 could be at least one of an ECG waveform, an EtCO2 waveform, an SpO2 waveform, temperature, invasive blood pressure, and non-invasive blood pressure. The third input of real-time sensor data 540 could be at least one of an ECG waveform, an EtCO2 waveform, an SpO2 waveform, temperature, invasive blood pressure, and non-invasive blood pressure. Finally, the physiological data displayed in the remaining vital sign channels could also be at least one of an ECG waveform, an EtCO2 waveform, an SpO2 waveform, temperature, invasive blood pressure, and non-invasive blood pressure. The data could be gathered by at least one of the sensors coupled to the external defibrillator, which typically gathers the aforementioned data.

In an example, the first vital sign channel 506 is displayed in a first position 544 at a top 546 of GUI 502, the second vital sign channel 508 is in a second position on the GUI 502, and the third vital sign channel 510 is in a third position on GUI 502. In some implementations the second vital sign channel 508 and the third vital sign channel 510 may configured to change positions. For example, the second vital sign channel 508 and the third vital sign channel 510 may be able to switch positions with each other. Alternatively, vital sign channels that are displaying ECG waveforms may be able to switch with each other.

In one aspect, a vital sign channel which displays an ECG waveform is always maintained in the first position 544 at the top 546 of GUI 502. In one example, the first input of real-time sensor data 536 in the first vital sign channel 506 is an ECG waveform, the second input of real-time sensor data 538 in the second vital sign channel 508 is an ECG waveform, and the third input of real-time sensor data 540 in the third vital sign channel 510 is an ECG waveform. In that example, any one of the first vital sign channel 506, the second vital sign channel 508, or the third vital sign channel 510 could be positioned in the first position 544 at the top 546 of the GUI 502. The same is true if more vital sign channels were added which displayed ECG waveforms. Any of the vital sign channels displaying an ECG waveform could be selected as the top vital sign channel on GUI 502. As long as there are more than one vital sign channel displaying an ECG waveform, users can reorder and place any of the ECG vital sign channels at the top of GUI 502.

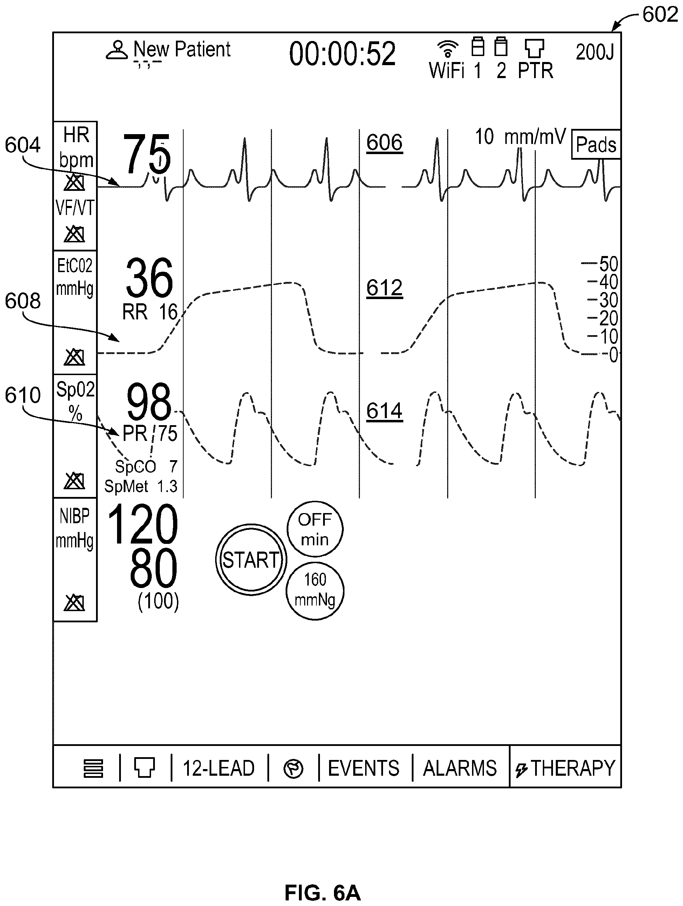

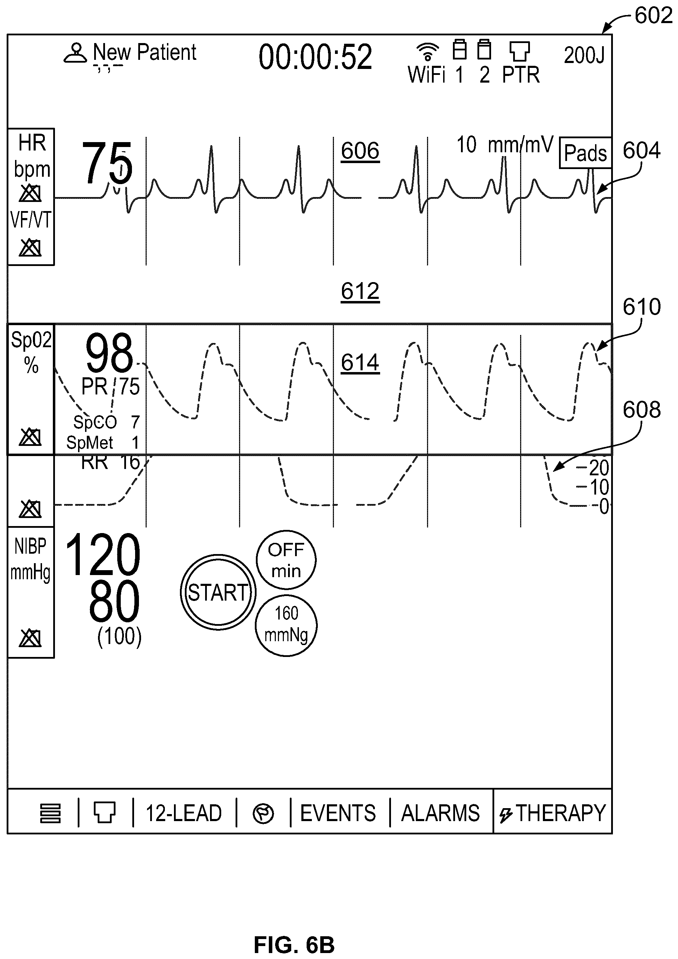

Figures 6A and 6B illustrate a GUI 602, such as the GUI 602 of Figure 4, that includes an example implementation of vital sign channel positions when only one vital sign channel displays an ECG waveform. In examples with only one vital sign channel displaying an ECG waveform, that vital sign channel should be the first vital sign channel 604 and must pinned to the top position on GUI 602. In an example aspect, the first vital sign channel 604 is dedicated to ECG waveforms. The remaining vital sign channels, which do not display ECG waveforms, could switch positions with each other. For example, when the first vital sign channel 604 displays an ECG waveform it may be pinned to the first position 606. When the second vital sign channel 608 and the third vital sign channel 610 display physiological data other than ECG waveforms, their positions could be moved by a user. Figure 6A illustrates that the second vital sign channel could start in the second position 612 and Figure 6B illustrates that the second vital channel could then be moved to the third position 614 on GUI 602. Similarly, Figure 6A illustrates that the third vital sign channel 610 could start in the third position 614 and Figure 6B illustrates that the third vital sign channel 610 could be moved in the second position 612on GUI 602.

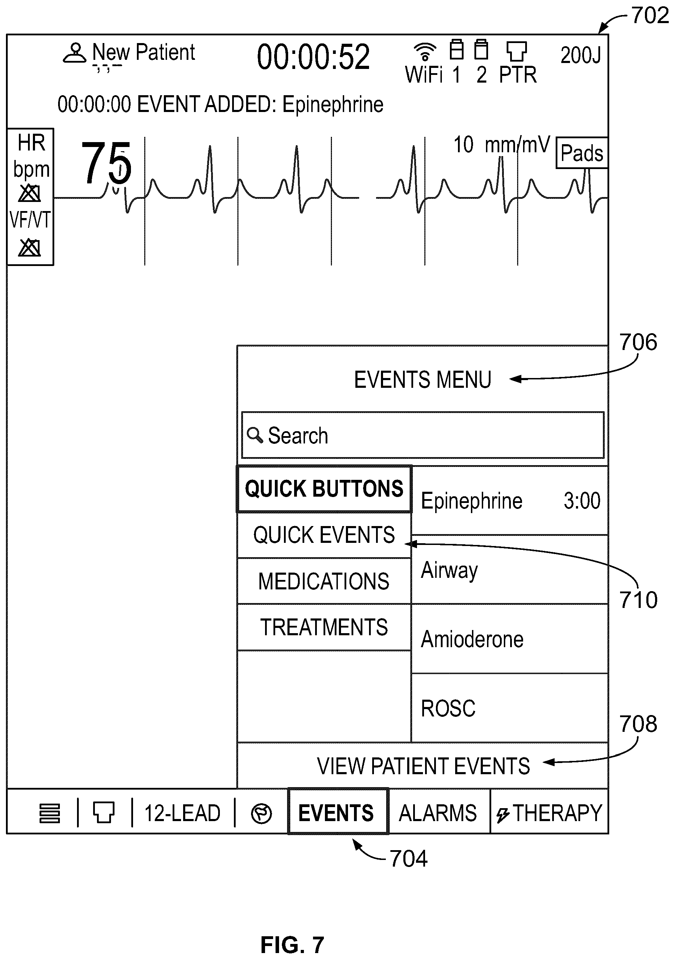

Figure 7 illustrates a GUI 702, such as the GUI 702 of Figure 4. The GUI 702 of the external defibrillator further includes at least one events menu button 704. An events menu 706 can be accessed by selecting an events menu button 704, as discussed previously in reference to Figure 4. Once the events menu button has been selected, the menu may appear on GUI 702. The events menu 706 may include a plurality of selectable options including an option to view a patient treatment event list 708, and an option to add an event 710.

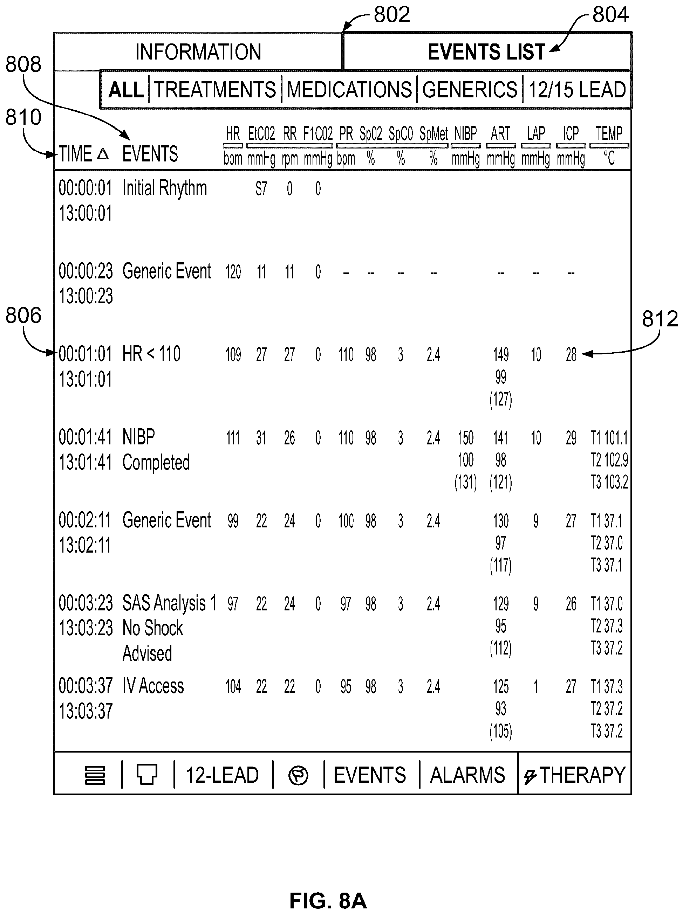

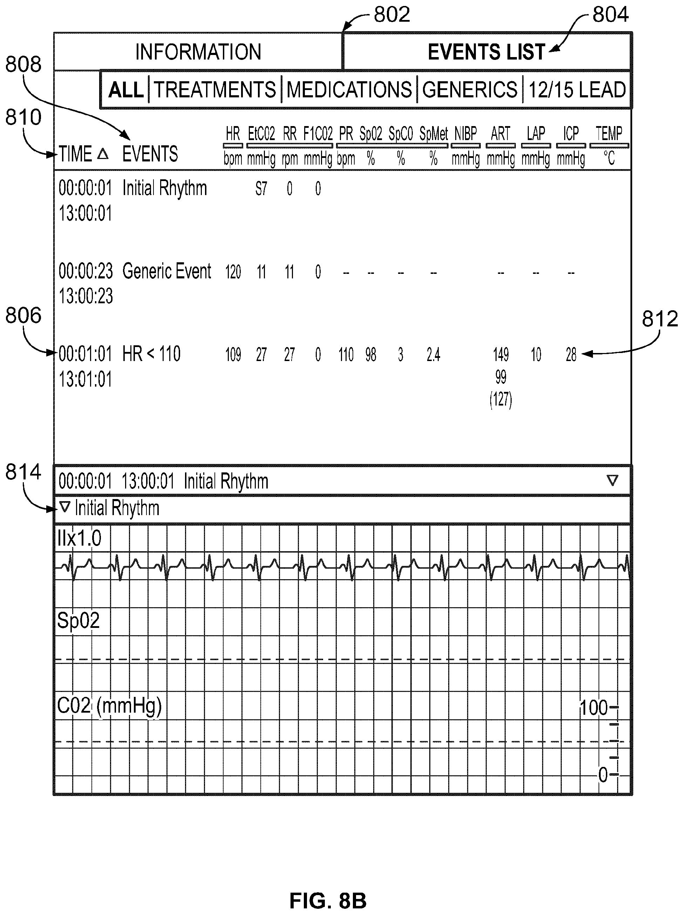

Figure 8A and 8B illustrate a GUI 802, such as the GUI of Figure 4, after selecting a menu option to view a patient treatment event list 804. As illustrated, the patient treatment event list 804 includes at least one reference to an event 806 in the first real-time sensor data. Particularly, the GUI 802 can include a list of events 808 (i.e., an event list) and times 810 associated with the events 808. The list of events 808 may be sorted in ascending time order from when treatment begins. Each event may also include patient information 812. The patient information may include a plurality of physiological data associated with the event. For example, the list 808 includes an event 806 of HR<100. For the event 806, there is a time at which it happened and values for heart rate, EtCO2, non-invasive blood pressure, invasive blood pressure, and temperature at the time the event occurred. Each event on the patient treatment event list 804 may further include a link to a waveform 814 associated with the event 806.

When the event 806 is selected, the link to the waveform 814 associated with the event 806 may be accessed. Figure 8B illustrates the GUI 802 when the link to the waveform 814 has been accessed. In Figure 8B, the link for the event HR <110 has been selected. The waveform 814 for the physiological data gathered using the sensors coupled to the external defibrillator may be displayed on GUI 802 for easy access.

### III. Example Methods

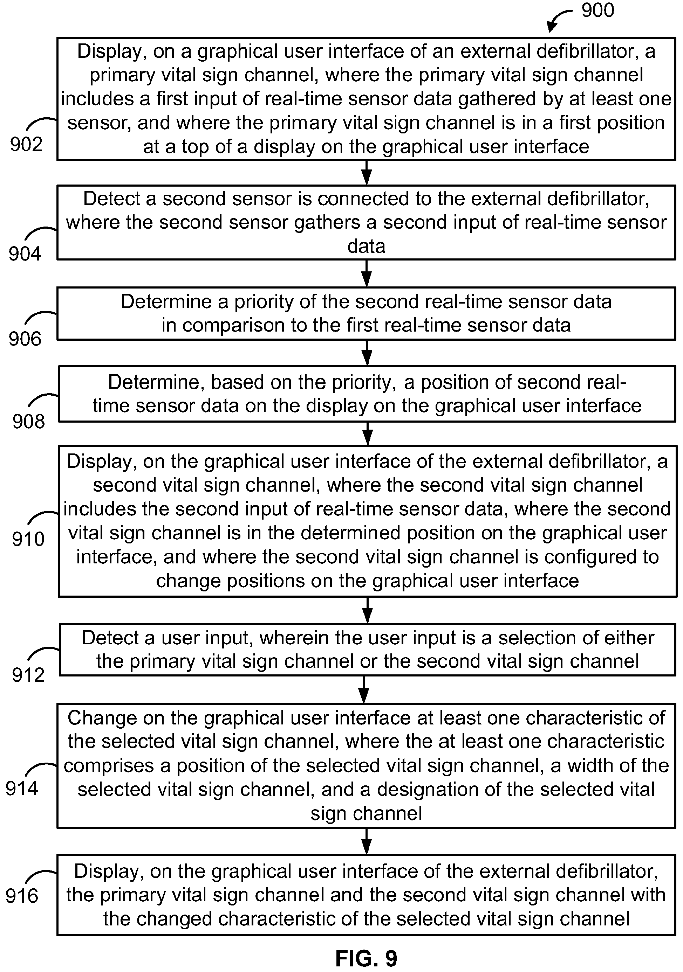

Figure 9 illustrates a flow chart of a method 900 for displaying vital sign channels on a GUI of an external defibrillator. The method 900 represents an example method that may include one or more operations, functions, or actions, as depicted by one or more of blocks 902-916, each of which may be performed by any of the external defibrillators or systems described herein. For instance, external defibrillator depicted in Figures 2, 3, and 4 may enable execution of method 900.

Those skilled in the art will understand that the flowchart of Figure 9 herein illustrates functionality and operations of certain implementations of the present disclosure. In this regard, each block of the flowchart may represent a module, a segment, or a portion of program code, which includes one or more instructions executable by one or more processors for implementing specific logical functions or steps in the process. The program code may be stored on any type of computer readable medium, for example, such as a storage device including a disk or hard drive.

In addition, each block may represent circuitry that is wired to perform the specific logical functions in the process. Alternative implementations are included within the scope of the example implementations of the present application in which functions may be executed out of order from that shown or discussed, including substantially concurrent or in reverse order, depending on the functionality involved, as would be understood by those reasonably skilled in the art.

When carrying out the method illustrated in Figure 9, the external defibrillator may display a first vital sign channel that includes real-time sensor data gathered by at least one sensor. When the external defibrillator detects a second sensor has been connected, the external defibrillator may determine a position to display the second real-time sensor data gathered by the second sensor based on the priorities of the sensor data. The second real-time sensor data could be displayed on GUI in a second vital sign channel along with the first vital sign channel. Even though the external defibrillator has displayed the vital sign channels in a position based on priority, a user can still change characteristics of the vital sign channels based on their personal preference. For example, the user could rearrange the order of the vital sign channels.

The method in Figure 9 may include displaying, on a GUI of an external defibrillator, a first vital sign channel, wherein the first vital sign channel comprises a first input of real-time sensor data gathered by at least one sensor, and wherein the first vital sign channel is in a first position on the GUI at block 902. At block 904, the method includes detecting a second sensor is connected to the external defibrillator, wherein the second sensor gathers a second input of real-time sensor data. At block 906, the method includes determining a priority of the second real-time sensor data in comparison to the first real-time sensor data. At block 908, the method includes determining, based on the priority, a position of second real-time sensor data on the GUI. At block 910, the method includes displaying, on the GUI of the external defibrillator, a second vital sign channel, wherein the second vital sign channel comprises the second input of real-time sensor data, wherein the second vital sign channel is in the determined position on the GUI, and wherein the second vital sign channel is configured to change positions on the GUI. At block 912, the method includes detecting a user input, wherein the user input is a selection of either the first vital sign channel or the second vital sign channel. At block 914, the method includes changing on the GUI at least one characteristic of the selected vital sign channel, wherein the at least one characteristic comprises a position of the selected vital sign channel, a width of the selected vital sign channel, and a designation of the selected vital sign channel. At block 916, the method includes displaying, on the GUI of the external defibrillator, the first vital sign channel and the second vital sign channel with the changed characteristic of the selected vital sign channel.

Block 902 may include displaying, on the GUI of the external defibrillator, a first vital sign channel. The first vital sign channel can include a first input of real-time sensor data gathered by at least one sensor. The first input of real-time sensor data on the first vital sign channel can be an ECG waveform, and may be in a first position at a top of the GUI. Since the first vital sign channel is an ECG waveform, the first vital sign channel may be permanently displayed on the GUI. At least one ECG waveform should be displayed on the GUI at all times. In an example implementation, the first vital sign channel may be pinned to the first position at the top of the GUI.

Block 904 may include detecting a second sensor has been connected to the external defibrillator. This detection may be based on electrical signals or impedance changes associated with the presence of a connected sensor. The external defibrillator may utilize standardized connectors and communication protocols, to recognize that the second sensor is compatible automatically. The second sensor may gather a second input of real-time sensor data. The second input of real-time sensor data could be any of an ECG waveform, an EtCO2 waveform, an SpO2 waveform, temperature, invasive blood pressure, and non-invasive blood pressure.

Block 906 may include determining a priority of the second real-time sensor data in comparison to the first real-time sensor data. The priorities may be automatically assigned based on what sensor data is deemed most relevant to a user of the external defibrillator. For example, ECG waveforms may always be considered the most relevant, so may have the highest priority. The data with the next priority may be invasive blood pressure, followed by EtCO2, followed by non-invasive blood pressure, followed by temperature. Within each data set, the data may be displayed on a first come, first come, first served basis. For example, different invasive blood pressures may be displayed based on the time the data was added to the GUI. The different parameters for invasive blood pressure to display on the GUI could be arterial blood pressure, pulmonary artery blood pressure, central venous pressure, intra cranial pressure, and left arterial pressure

Block 908 may include determining, based on the priority, a position of second real-time sensor data on the GUI. The higher the priority of the data, it will be displayed in a vital sign channel closer to the top of the GUI.

In one example, if there are five vital sign channels displaying ECG waveforms, and other sensors are connected that provide real-time sensor data other than ECG data, the vital sign channels displaying the real-time sensor data for the new sensors connected may automatically replace the lowest vital sign channel displaying an ECG waveform. However, the first vital sign channel displaying the ECG waveform will always remain on the GUI.

Block 910 can include displaying, on the GUI of the external defibrillator, a second vital sign channel. The second vital sign channel can include the second input of real-time sensor data and may be displayed in the determined position on the GUI. However, the second vital sign channel may be configured to change positions on the GUI. For example, the position of the second vital sign channel could change based on priority, or the user can drag and drop the second vital sign channel to a new position on the GUI. One aspect can include determining the priority of the second input of real-time sensor data is higher than a priority of the first input of real-time sensor data. In response the GUI of the external defibrillator can display the second vital sign channel in the first position on the GUI, and display the first vital sign channel in a second position on the GUI. Alternatively, if the priority of the first real-time sensor data was higher than the priority of the second real-time sensor data, the GUI of the external defibrillator can display the first vital sign channel in the first position on the GUI, and display the second vital sign channel in the second position on the GUI.

Additional implementations can include reprioritizing data as additional sensors are connected to the external defibrillator. The method can include detecting a third sensor is connected to the external defibrillator. The third sensor can gather a third input of real-time sensor data. The method can include determining a priority of the third input of real-time sensor data in comparison to the first input of real-time sensor data and the second input of real-time sensor data, and determining, based on the priority, a position of third real-time sensor data on the GUI. Based on that, the method can include determining reprioritized positions for the first real-time sensor data and the second real-time sensor data. The GUI of the external defibrillator can then display a third vital sign channel in its determined position on the GUI. The third vital sign channel can include the third input of real-time sensor data and can be configured to change positions on the GUI. The GUI can also display the first vital sign channel and the second vital sign channel in the reprioritized positions on the GUI. Waveforms and vital sign channels can be continuously reprioritized as additional sensors are plugged into the external defibrillator.

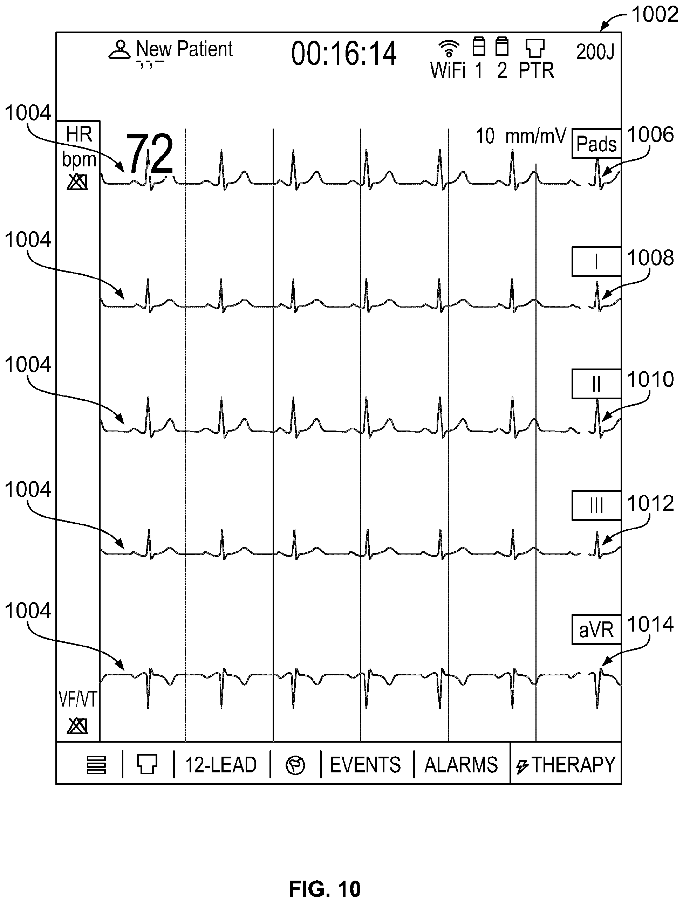

Figure 10 illustrates an example of the GUI 1002 of the external defibrillator displaying up to five ECG waveforms 1004. One ECG waveform in each of the first vital sign channel 1006, the second vital sign channel 1008, the third vital sign channel 1010 and the fifth vital sign channel 1012. No other type of real-time sensor data is displayed. Either no other sensors have been connected, or the vital sign channels displaying the other type of real-time sensor data has been removed, such as by a user. If a second sensor was connected and the real-time sensor data was not an ECG waveform, the real-time sensor data from the second sensor would replace the ECG waveform in the fifth vital sign channel. If a third sensor was connected and the real-time sensor data was not an ECG waveform, the real-time sensor data from the third sensor would replace the ECG waveform in either the fourth or fifth vital sign channel, depending on the relative priority of the real-time sensor data from the second and third sensors. As additional sensors are connected, or additional real-time data is added to the GUI 1002, this process would continue until the ECG waveforms in the second and third vital sign channels are also replaced with the sensor data ordered in the various vital sign channels based on priority. The ECG waveform in the first vital sign channel would not be replaced.

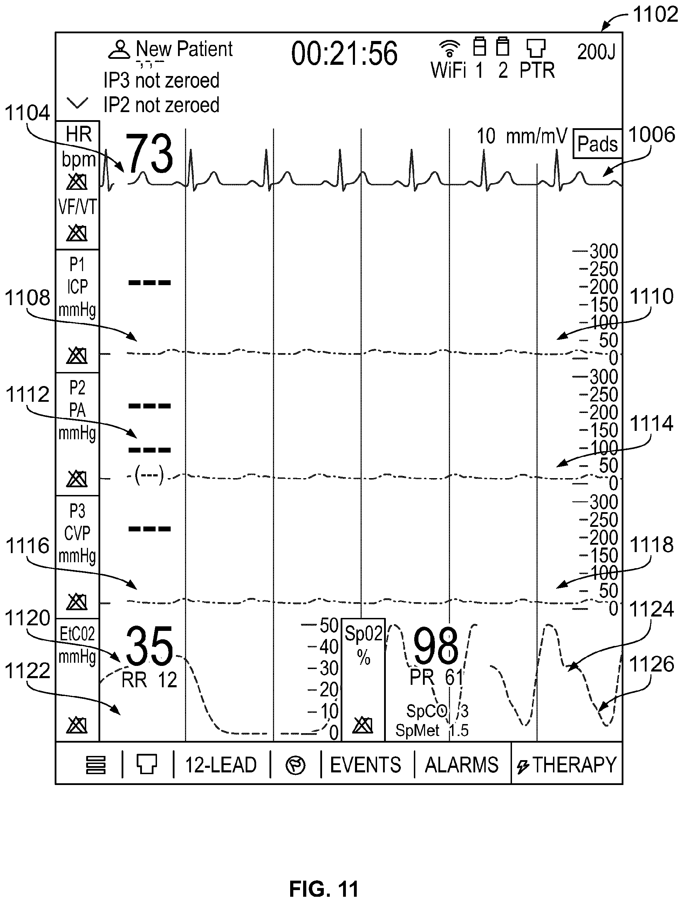

Figure 11 illustrates an example of the GUI 1102 of the external defibrillator displaying up to one ECG waveform 1104 in the first vital sign channel 1106. Arterial blood pressure 1108 is displayed in the second vital sign channel 1110. Pulmonary artery blood pressure 1112 is displayed in the third vital sign channel 1114. Central venous pressure 1116 is displayed in the fourth vital sign channel 1118. EtCO2 1120 is displayed in the fifth vital sign channel 1122. SpO2 1124 is displayed in the sixth vital sign channel 1126. In order to display both EtCO2 and SpO2 in the last row available on the GUI 1102, the fifth vital waveform channel 1122 and the sixth vital waveform channel 1126 are automatically displayed at half width. Because EtCO2 and SpO2 have the lowest priority, they are displayed in the bottom position on GUI 1102 and their widths are reduced so that more real-time sensor data can be displayed.

In some implementations, any of the vital sign channels can include a trend graph. The trend graph can include a bar graph of the trends in the real-time sensor data. A trend graph can only be added if there is available space within a channel. If there are no available rows, a user may have to make space (i.e., remove a parameter, close a different trend graph) and then add the trend graph. In general, vital sign channels have priority over trend channels, so if a real-time sensor data is detected and needs the channel, the external defibrillator will close the trend graph closest to the bottom of the GUI.

Vital sign channels that display ECG waveforms could include trend graphs for heart rate and/or STJ. Vital sign channels that display EtCO2 could include trend graphs for respiratory rate and/or CO2. Vital sign channels that display SpCO2 could include trend graphs for Sp02, SpCO, SpMet, and/or Pulse. Vital sign channels that display invasive blood pressure could include a trend graph for arterial blood pressure.

Block 912 can include detecting a user input. The user input can be an instruction to add or remove a vital sign channel. Alternatively, the user input can be a selection of any of the vital sign channels displayed on the GUI, for example either the first vital sign channel or the second vital sign channel. Detecting the user input can include detecting a touch drag and drop motion across the GUI. The touch drag and drop motion can include changing the position of the selected vital sign channel. Users can reorder vital sign channels using select/deselect controls as well as the drag-and-move operations. Dragging the channel up or down the screen moves other channels automatically making room for repositioning the dragged channel. Releasing the touch press will "drop" the channel. Figures 6A and 6B, as previously described, illustrate dragging and dropping a vital sign channel to change the vital sign channel's position.

Block 914 can include changing on the GUI at least one characteristic of the selected vital sign channel. As previously described, the at least one characteristic could be the position of the selected vital sign channel. Alternatively, the at least one characteristic could a width of the selected vital sign channel. For example, if the user input was to add a vital sign channel, the width of one of the vital sign channels may need to narrow in order to make room in a vital sign channel. If real-time sensor data was automatically removed based on priority, a user could choose to add it back in, which would automatically cause width compression of the lowest priority real-time sensor data. Finally, the at least one characteristic could be a designation of the selected vital sign channel. The designation of the selected vital sign channel could be to make a vital sign channel displaying an ECG waveform the "primary" vital sign channel.

Block 916 can include displaying, on the GUI of the external defibrillator, the first vital sign channel and the second vital sign channel with the changed characteristic of the selected vital sign channel.

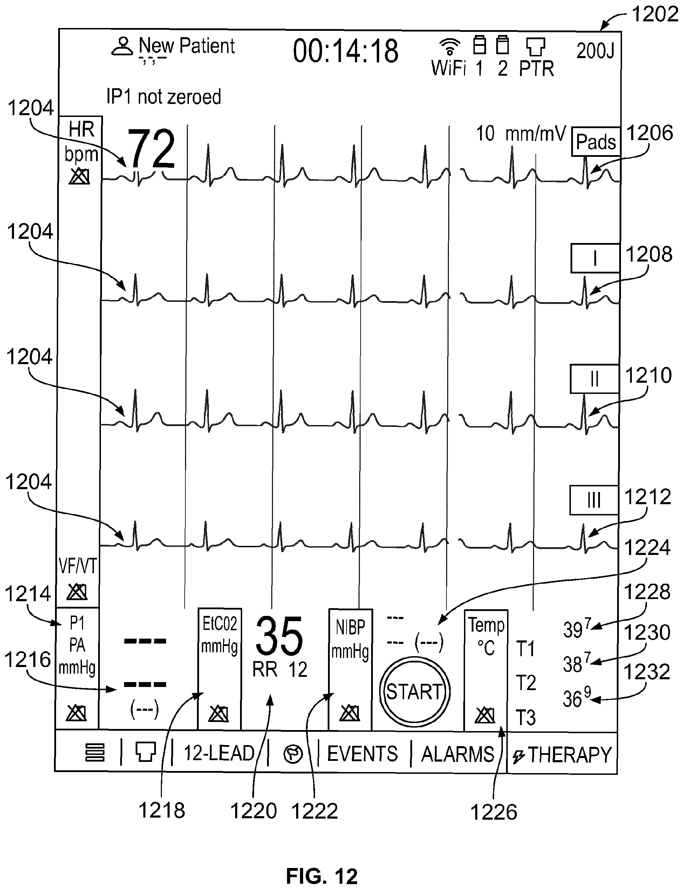

Figure 12 illustrates an example of the GUI 1202 of the external defibrillator displaying an ECG waveform 1204 in each of the first vital sign channel 1206, the second vital sign channel 1208, the third vital sign channel 1210 and the fourth vital sign channel respectively 1212. Pulmonary artery blood pressure 1214 is displayed in the fifth vital sign channel 1216. EtCO2 1218 is displayed in the sixth vital sign channel1220. Non-invasive blood pressure 1222 is displayed in the seventh vital sign channel 1224. Temperature 1226 is displayed in seventh vital sign channel 1228, eighth vital sign channel 1230, and ninth vital sign channel 1232. In order to display Pulmonary Artery blood pressure, EtCO2, Non-invasive blood pressure, and temperature in the last row, they must be displayed as quarter widths. The temperature parameters are able to stack within a quarter width, so three different vital sign channels with different real-time data can be displayed. This implementation may be the result of adding ECG waveforms back in after the external defibrillator had automatically removed them when new sensors were detected. Vital sign channels can collapse their width in order to display more vital sign channels on the GUI 1202.

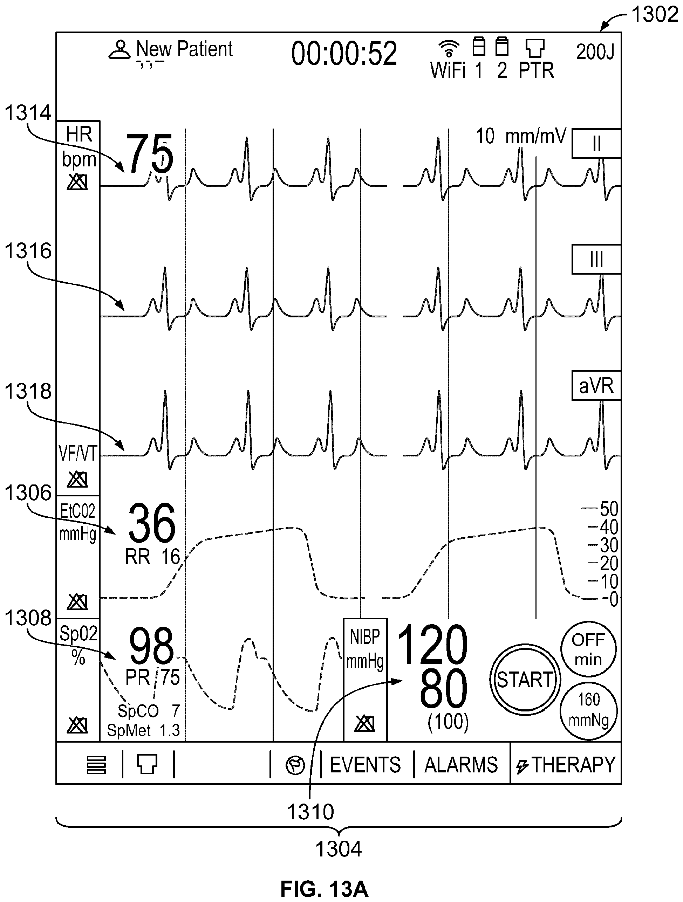

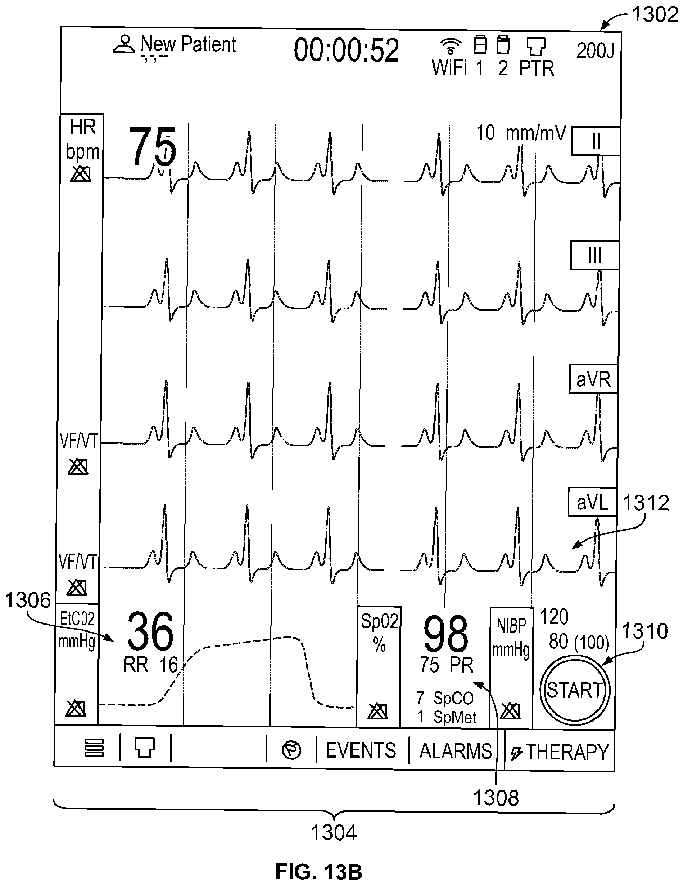

For example, Figure 13A and 13B illustrate how on a GUI 1302 the width 1304 of the fourth vital sign channel for EtCO2 1306, the fifth vital sign channel for SpO2 1308, and the sixth vital sign channel for non-invasive blood pressure 1310 must be reduced in order to add an additional vital sign channel displaying an ECG waveform 1312. In Figure 13A there are a first vital sign channel 1314, a second vital sign channel 1316, and a third vital sign channel 1318 all of which can display ECG waveforms at full width, a fourth vital sign channel 1306 displaying EtCO2 at full width, a fifth vital sign channel 1308 displaying SpO2 at half width, and a sixth vital sign channel 1310 displaying non-invasive blood pressure at half width. In Figure 13B, once the additional vital sign channel 1312 was added to display a fourth ECG waveform, the fourth vital sign channel 1306 displaying EtCO2 reduced to half width, the fifth vital sign channel 1308 displaying SpO2 reduced to quarter width, and the sixth vital sign channel 1310 displaying non-invasive blood pressure reduced to quarter width.

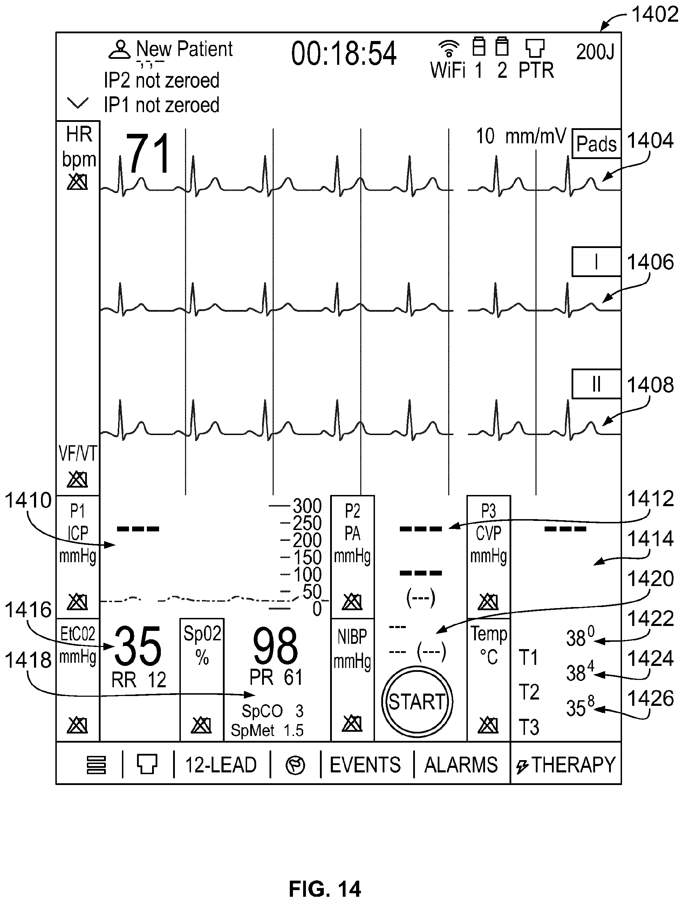

Figure 14 illustrates an example of a GUI 1402 on an external defibrillator displaying the twelve vital sign channels. ECG waveforms are displayed in the first vital sign channel 1404, the second vital sign channel 1406, and the third vital sign channel 1408. Intra cranial pressure is displayed in the fourth vital sign channel 1410. Pulmonary artery blood pressure is displayed in the fifth vital sign channel 1412. Central venous pressure is displayed in the sixth channel 1414. EtCO2 is displayed in the seventh vital sign channel 1416. SpO2 is displayed in the eighth vital sign channel 1418. Non-invasive blood pressure is displayed in the ninth vital sign channel 1420. Temperature is displayed in tenth vital sign channel 1422, eleventh vital sign channel 1424, and twelfth vital sign channel 1426.

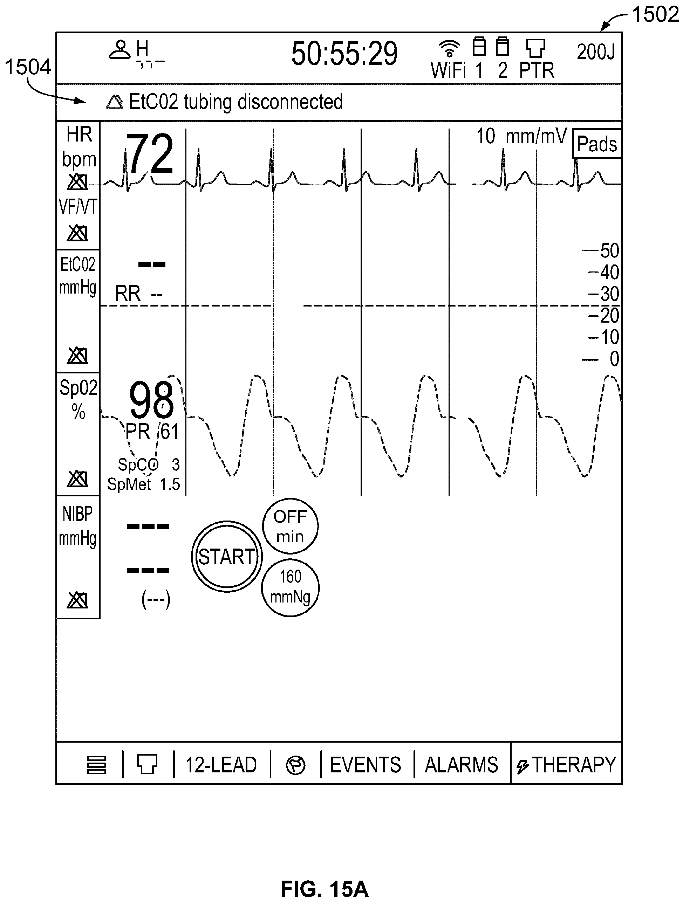

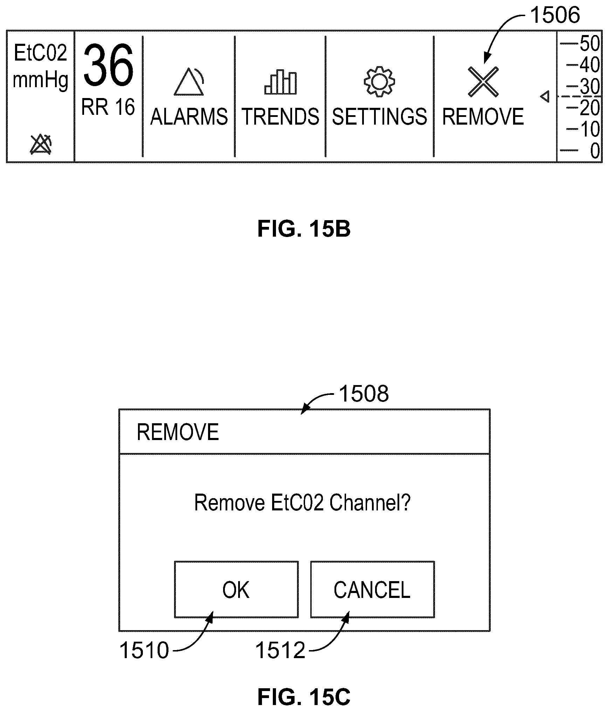

An example of the method may further include detecting that the second sensor has been removed from the external defibrillator. Figure 15A illustrates a GUI 1502 of the external defibrillator. Once the external defibrillator has detected that the second sensor was removed, the method may include displaying an alert 1504 that the second sensor was removed from the external defibrillator. For example, the alert may state that sensor tubing was disconnected. Figure 15B illustrates a portion of GUI 1502. The method may further include detecting that a remove option 1506 has been selected via the GUI for the second vital sign channel. The remove option 1506 may be displayed in the vital sign channel that previously included the real-time sensor data, along with a settings option, a trend graph option, and an alarm option. In response to detecting that the user selected the remove option 1506 for the second vital sign channel, the method may include displaying a dialog confirmation 1508 to remove the second vital sign channel. Figure 15C illustrates the dialog box 1508. The dialog confirmation 1508 may include a question of whether to remove the vital sign channel with a confirmation 1510 or a denial 1512. The method can further include detecting that the dialog confirmation was selected via the GUI 1502. Based on receiving the confirmation, the method can include removing the second vital sign channel from the GUI 1502. In the case where the denial was selected, the dialog confirmation may go away and the GUI 1502 may display the vital sign channel with the remove option, the settings option, the trend graph option, and the alarm option. This method is to make sure that real-time sensor data is not lost if a sensor was accidentally disconnected from the external defibrillator. If the sensor is re-coupled to the external defibrillator, the real-time sensor data automatically re-appears in the vital sign channel

Once a vital sign channel is removed, the remaining vital sign channels may expand their widths to fill in the space left. For example, all of the remaining vital sign channels below the vital sign channel that was removed will be shifted up.

Figure 15 illustrates when EtCO2 tubing was disconnected, however the method would operate the same with any other type of sensor, except the last sensor associated with the only ECG waveform on the GUI.

The method may further include displaying a menu on the GUI. The menu on the GUI may be the menu previously illustrated in Figure 7. The menu can include selectable options for a patient treatment event list and adding an event.

In an example, the external defibrillator automatically logs events regarding vital sign monitoring and therapy in the patient treatment event list. The method can include monitoring vital signs via the at least one sensor coupled to the external defibrillator and determining, based on data from the at least one sensor, a vital sign event. The vital sign event can include at least one of a shock, a pacing set, pacing stopped, pacing started, pacing changed, pacing paused, an alarm violation, print, and 12/15 lead. The external defibrillator can add the vital sign event to the patient treatment event list. The patient treatment event list provides access to a list of waveform events that occurred during treatment of the patient.

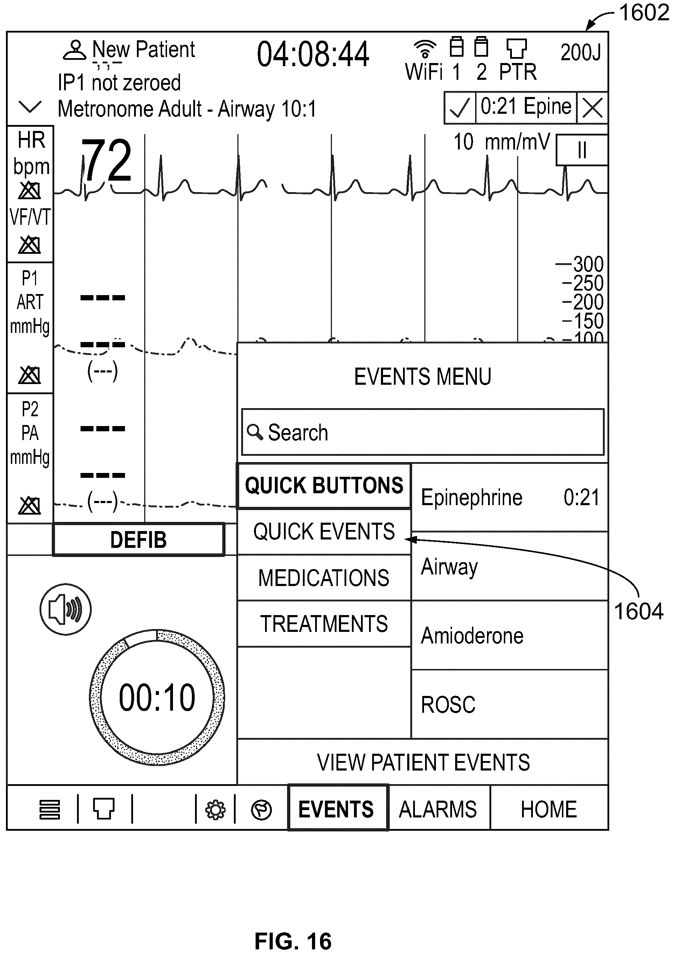

The method can also include detecting a user request to add an event to the patient treatment event list. The event comprises at least one of a medication administration and a treatment. Figure 16 illustrates a GUI 1602 for a user adding an event 1604 of administering epinephrine to a patient. An example implementation can also include a timed drug feature. Epinephrine and some other drugs need to be repeated in certain period. This feature is a timer, and when default count down time is reached, an alert is displayed on the GUI to either give another episode or dismiss.

The method can further include detecting a selection of the patient treatment event list from a menu, and displaying, on the GUI of the external defibrillator, the patient treatment event list. As previously mentioned, the patient treatment event list can include patient information, a vital signs events list, and a link to a waveform associated with the vital sign event. The patient treatment event list will include automatically logged events and user added events. When an event is selected in the events list, the waveform viewer opens at the bottom of the event list, displaying waveform data associated with that event.

The patient treatment event list can have a header area with time sorting and filter controls. The method can further include sorting the patient treatment event list based on the time that the event occurred. Sorting events by time can order the events in either ascending or descending time order since the start of the current patient episode. The current patient episode may be medical event that is occurring for which medical intervention is needed. The filter controls provide filtering by event type: ALL, TREATMENTS, MEDICATIONS, GENERIC EVENTS and 12/15-LEAD.

In still another aspect, a non-transitory computer-readable medium storing instructions is disclosed that, when the instructions are executed by one or more processors, causes the one or more processors to perform operations for displaying vital sign channels in an order which the user can rearrange based on preference. The operations may include displaying, on a GUI of an external defibrillator, a first vital sign channel. The first vital sign channel may include a first input of real-time sensor data gathered by at least one sensor, and may be in a first position on the GUI. The operations may also include detecting a second sensor is connected to the external defibrillator. The second sensor can gather a second input of real-time sensor data. The operations may further include determining a priority of the second real-time sensor data in comparison to the first real-time sensor data. The operations may also include determining, based on the priority, a position of second real-time sensor data on the GUI. The operations may further include displaying, on the GUI, a second vital sign channel in the determined position on the GUI. The second vital sign channel can include the second input of real-time sensor data. The second vital sign channel may also be configured to change positions on the GUI. The operations can also include detecting a user input. The user input can be a selection of either the first vital sign channel or the second vital sign channel. The operations can further include changing on the GUI at least one characteristic of the selected vital sign channel. The at least one characteristic can include a position of the selected vital sign channel, a width of the selected vital sign channel, and a designation of the selected vital sign channel. The operations can also include displaying, on the GUI, the first vital sign channel and the second vital sign channel with the changed characteristic of the selected vital sign channel.

In an example, the operations may also include automatically utilizing how the first vital sign channel, the second vital sign channel, a third vital sign channel, a fourth vital sign channel, a fifth vital sign channel, and a sixth vital sign channel are displayed on the GUI. Utilizing how the vital sign channels are displayed may include displaying the vital sign channels in positions based on priority. Furthermore, utilizing the display may include collapsing the widths of low priority vital sign channels so that more vital sign channels can be fit on the GUI, as in the manner previously described.

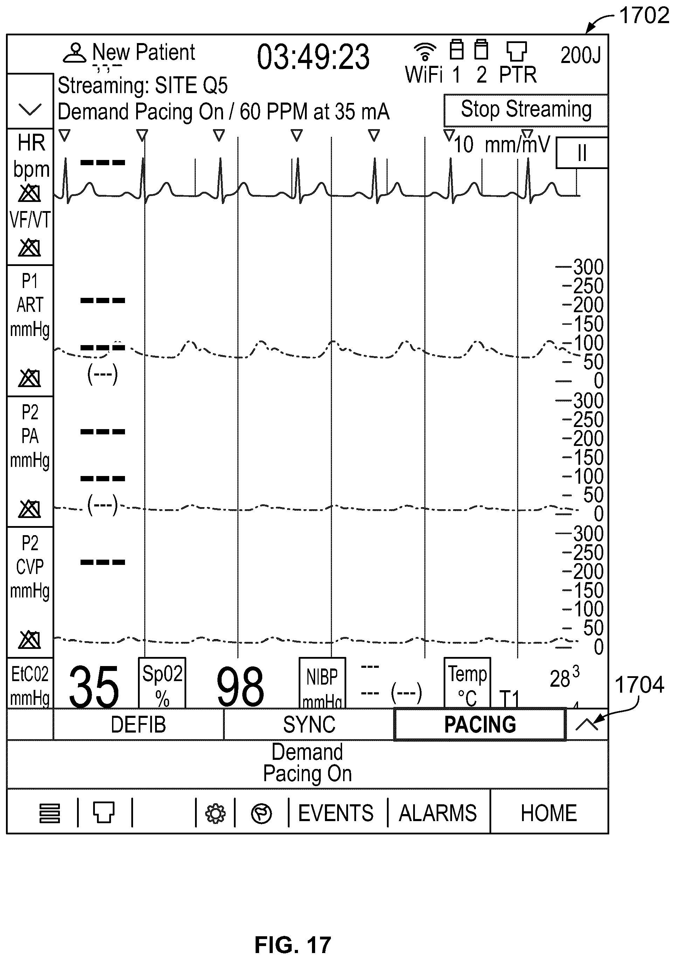

Another example can include collapsing therapy windows while manual therapy is still active in order to monitor the screen of the GUI. When the therapy overlay is up, it can block vital sign channels. However, by selecting a minimize/maximize feature on the therapy overlay via the GUI, the vital sign channels can become visible again. Figure 17 illustrates a GUI 1702 in active therapy. The minimize/maximize feature 1704 is an arrow on the bottom of the GUI 1702. When the therapy window is collapsed, a user can select the DEFIB, SYNC, or PACING tab, or you press charge hard key, to automatically expand the therapy window to take care higher priority therapy issues.

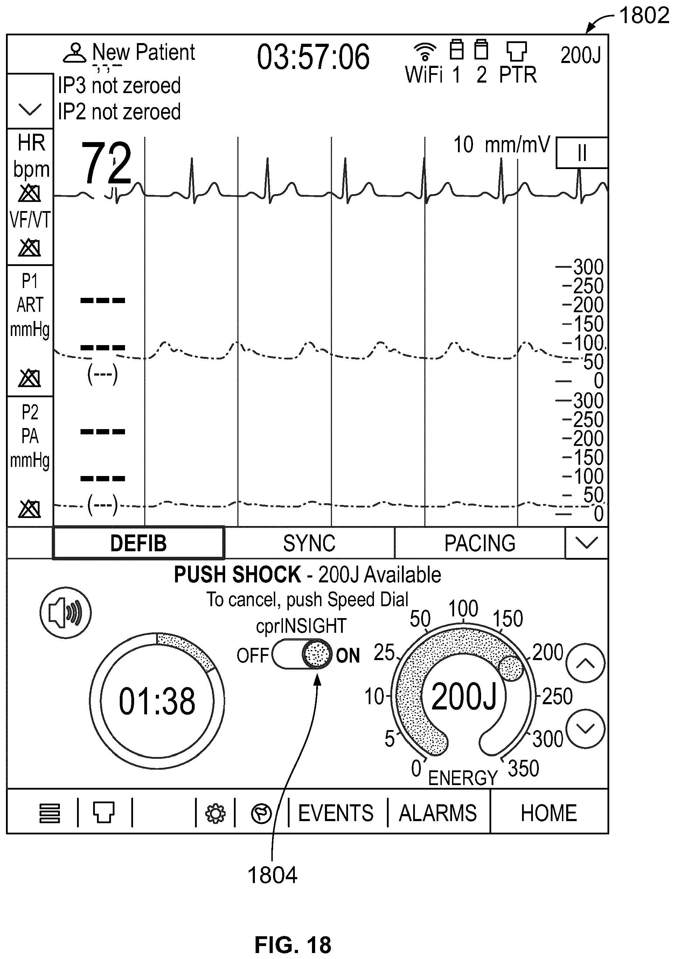

In the therapy window, a user can also control CPRInsight by turning it off or on as illustrated in Figure 18 via an on off toggle 1804 on GUI 1802.

Further, the present application includes enabling a processor or user to add, delete change positions, or otherwise change characteristics of the vital sign channels displayed on a GUI that is independent of a defibrillator. The sensor data that is being, has been or could be displayed on a defibrillator GUI could be obtained in the same manner as described above, to be displayed on the GUI that is independent from the defibrillator. This would include, for example, a tablet, smartphone, laptop or desktop computer having a GUI that is displaying such sensor data and includes both real-time and post event display. The sensor data may be obtained in the same manner described above, and transmitted to the GUI that is independent from the defibrillator.

Sensor data could be transmitted from the external defibrillator to the independent GUI through wireless communication protocols such as Bluetooth or Wi-Fi. As previously mentioned, the defibrillator may be equipped with a transmitter capable of securely sending real-time physiological data, including electrocardiogram (ECG) information and device status, to the independent GUI. The GUI would have a corresponding receiver to capture and interpret the transmitted data. The sensor data could then be displayed on the independent GUI in the same manner described above.

The foregoing summary is illustrative only and is not intended to be in any way limiting. In addition to the illustrative aspects, implementations, and features described above, further aspects, implementations, and features will become apparent by reference to the figures and the following detailed description.

## Claims

1. A method (900) comprising:
displaying (902), on a GUI of an external defibrillator, a first vital sign channel, wherein the first vital sign channel comprises a first input of real-time sensor data gathered by at least one sensor, and wherein the first vital sign channel is in a first position on the GUI;
detecting (904) a second sensor is connected to the external defibrillator, wherein the second sensor gathers a second input of real-time sensor data;
determining (906) a priority of the second input of real-time sensor data in comparison to the first input of real-time sensor data;
determining (908), based on the priority, a position of second real-time sensor data on the GUI;
displaying (910), on the GUI of the external defibrillator, a second vital sign channel, wherein the second vital sign channel comprises the second input of real-time sensor data, wherein the second vital sign channel is in the determined position on the GUI, and wherein the second vital sign channel is configured to change positions on the GUI;
detecting (912) a user input, wherein the user input is a selection of either the first vital sign channel or the second vital sign channel;
changing (914) on the GUI at least one characteristic of the selected vital sign channel, wherein the at least one characteristic comprises a position of the selected vital sign channel, a width of the selected vital sign channel, and a designation of the selected vital sign channel;
displaying (916), on the GUI of the external defibrillator, the first vital sign channel and the second vital sign channel with the changed characteristic of the selected vital sign channel;
detecting that the second sensor has been removed from the external defibrillator;
displaying an alert that the second sensor was removed from the external defibrillator;
detecting that a remove option has been selected via the GUI for the second vital sign channel;
in response to detecting that the remove option was selected for the second vital sign channel, displaying a dialog confirmation to remove the second vital sign channel;
detecting that the dialog confirmation was selected via the GUI; and
removing the second vital sign channel from the GUI.

2. The method (900) of claim 1, further comprising:
determining the priority of the second input of real-time sensor data is higher than a priority of the first input of real-time sensor data;
displaying the second vital sign channel in a position above the first vital sign channel; and
displaying the first vital sign channel in a second position on the GUI.

3. The method (900) of claim 1 or 2, further comprising:
detecting a third sensor is connected to the external defibrillator, wherein the third sensor gathers a third input of real-time sensor data;
determining a priority of the third input of real-time sensor data in comparison to the first input of real-time sensor data and the second input of real-time sensor data;
determining, based on the priority, a position of third real-time sensor data on the GUI;
determining reprioritized positions for the first real-time sensor data and the second real-time sensor data;
displaying, on the GUI of the external defibrillator, a third vital sign channel, wherein the third vital sign channel comprises the third input of real-time sensor data, wherein the third vital sign channel is in the determined position on the GUI, and wherein the third vital sign channel is configured to change positions on the GUI; and
displaying the first vital sign channel and the second vital sign channel in the reprioritized positions on the GUI.

4. The method (900) of any of claims 1-3, wherein the first input of real-time sensor data on the first vital sign channel is an ECG waveform, wherein the first vital sign channel is permanently displayed on the GUI, and wherein the first vital sign channel is pinned to the first position at the top of the GUI.

5. The method (900) of any of claims 1-4, wherein detecting the user input further comprises detecting a touch drag and drop motion across the GUI, and wherein the touch drag and drop motion comprises changing the position of the selected vital sign channel.

6. The method (900) of any of claims 1-5, further comprising displaying, on the GUI, a menu, wherein the menu comprises selectable options for a patient treatment event list and adding an event.

7. The method (900) of claim 6, further comprising:
a) monitoring vital signs via the at least one sensor coupled to the external defibrillator;
determining, based on data from the at least one sensor, a vital sign event, wherein the vital sign event comprises at least one of a shock, a pacing set, pacing stopped, pacing started, pacing changed, pacing paused, an alarm violation, print, and 12/15 lead;
adding the vital sign event to the patient treatment event list;
detecting a selection of the patient treatment event list from a menu; and
displaying, on the GUI of the external defibrillator, the patient treatment event list, wherein the patient treatment event list comprises patient information, a vital signs events list, and a link to a waveform associated with the vital sign event; and/or
b) detecting a user request to add an event to the patient treatment event list, wherein the event comprises at least one of a medication administration and a treatment; and/or
c) sorting the patient treatment event list based on the time that the event occurred, wherein the sorting is in either ascending or descending order since a start of a current patient episode; and/or
d) filtering the patient treatment event list based on at least one of treatments, medications, generic events, and 12/15 lead.

8. An external defibrillator (102, 202, 302) comprising:
at least one sensor (118) removably coupled to the external defibrillator;
a GUI (402) configured to display a plurality of patient data, wherein the GUI comprises:
a first vital sign channel (420), wherein the first vital sign channel comprises a first input of real-time sensor data (464) gathered by the at least one sensor, and wherein the first vital sign channel is in a first position (454) on the GUI;
a second vital sign channel (450), wherein the second vital sign channel comprises a second input of real-time sensor data (468) gathered by the at least one sensor, wherein the second vital sign channel is in a second position (460) on the GUI, and wherein the second vital sign channel is configured to change positions;
a third vital sign channel (452), wherein the third vital sign channel comprises a third input of real-time sensor data (470) gathered by the at least one sensor, wherein the third vital sign channel is in a third position (462) on the GUI, and wherein the third vital sign channel is configured to change positions; and
a menu, wherein the menu comprises a plurality of selectable options including a patient treatment event list, and wherein the patient treatment event list includes at least one reference to an event in the first input of real-time sensor data;
**characterized in that** the external defibrillator further comprises a processor configured to perform the method according to any of the preceding claims.

9. The external defibrillator of claim 8, wherein the first input of real-time sensor data (464) is an ECG waveform.

10. The external defibrillator of claim 8 or 9, wherein the first vital sign channel (420) is pinned to the first position (454), the second vital sign channel (450) is in the third position (460), and the third vital sign channel (452) is in the second position (462).

11. The external defibrillator of claim 8, 9 or 10, wherein the first input of real-time sensor data (464) is at least one of an ECG waveform, an EtCO2 waveform, an SpO2 waveform, temperature, heart rate, carboxyhemoglobin concentration, methemogobin concentration, respiration rate, CO2, invasive blood pressure, and non-invasive blood pressure, wherein the second input of real-time sensor data (468) is at least one of an ECG waveform, an EtCO2 waveform, an SpO2 waveform, temperature, invasive blood pressure, and non-invasive blood pressure, and wherein the third input of real-time sensor data (470) is at least one of an ECG waveform, an EtCO2 waveform, an SpO2 waveform, temperature, invasive blood pressure, and non-invasive blood pressure, for instance wherein the first input of real-time sensor data (464) is an ECG waveform, the second input of real-time sensor data (468) is an ECG waveform, and the third input of real-time sensor data (470) is an ECG waveform, and wherein one of the first vital sign channel (420), the second vital sign channel (450), or the third vital sign channel (452) is in the first position (454) at the top of the GUI.

12. The external defibrillator of any of claims 8-11, wherein each of the first vital sign channel (420), the second vital sign channel (450), and the third vital sign channel (452) comprise a width, and wherein the width is at least one of full width, half width, and quarter width,
wherein optionally, at full width, the first vital sign channel (420), the second vital sign channel (450), and the third vital sign channel (452) include a trend, and half width or quarter width the first vital sign channel (420), the second vital sign channel (450), and the third vital sign channel (452) do not include the trend; and/or
wherein the patient treatment event list further comprises patient information, an events list, and a link to a waveform associated with the event, and wherein the events list is sorted by time order.

13. A non-transitory computer-readable medium having computer-executable instructions stored thereon that, if executed by one or more processors of an external defibrillator, cause the processors to perform operations, comprising:
displaying, on a GUI of the external defibrillator, a first vital sign channel, wherein the first vital sign channel comprises a first input of real-time sensor data gathered by at least one sensor, and wherein the first vital sign channel is in a first position on the GUI;
detecting a second sensor is connected to the external defibrillator, wherein the second sensor gathers a second input of real-time sensor data;
determining a priority of the second input of real-time sensor data in comparison to the first input of real-time sensor data;
determining, based on the priority, a position of second real-time sensor data on the GUI;
displaying, on the GUI of the external defibrillator, a second vital sign channel, wherein the second vital sign channel comprises the second input of real-time sensor data, wherein the second vital sign channel is in the determined position on the GUI, and wherein the second vital sign channel is configured to change positions on the GUI;
detecting a user input, wherein the user input is a selection of either the first vital sign channel or the second vital sign channel;
changing on the GUI at least one characteristic of the selected vital sign channel, wherein the at least one characteristic comprises a position of the selected vital sign channel, a width of the selected vital sign channel, and a designation of the selected vital sign channel;
displaying, on the GUI of the external defibrillator, the first vital sign channel and the second vital sign channel with the changed characteristic of the selected vital sign channel;
detecting that the second sensor has been removed from the external defibrillator;
displaying an alert that the second sensor was removed from the external defibrillator;
detecting that a remove option has been selected via the GUI for the second vital sign channel;
in response to detecting that the remove option was selected for the second vital sign channel, displaying a dialog confirmation to remove the second vital sign channel;
detecting that the dialog confirmation was selected via the GUI; and
removing the second vital sign channel from the GUI.

14. The non-transitory computer-readable medium of claim 13, wherein the operations further comprise automatically utilizing displaying the first vital sign channel, the second vital sign channel, a third vital sign channel, a fourth vital sign channel, a fifth vital sign channel, and a sixth vital sign channel on the GUI; and/or
wherein the first input of real-time sensor data on the first vital sign channel is an ECG waveform, wherein the first vital sign channel is permanently displayed on the GUI, and wherein the first vital sign channel is pinned to the first position at the top of the GUI.

## Patentansprüche

1. Verfahren (900), das Folgendes umfasst:
Anzeigen (902), auf einer GUI eines externen Defibrillators, eines ersten Vitalzeichenkanals, wobei der erste Vitalzeichenkanal eine erste Eingabe von Echtzeit-Sensordaten umfasst, die von mindestens einem Sensor gesammelt werden, und wobei sich der erste Vitalzeichenkanal an einer ersten Position auf der GUI befindet;
Erfassen (904), dass ein zweiter Sensor mit dem externen Defibrillator verbunden ist, wobei der zweite Sensor eine zweite Eingabe von Echtzeit-Sensordaten sammelt;
Bestimmen (906) einer Priorität der zweiten Eingabe von Echtzeit-Sensordaten im Vergleich zu der ersten Eingabe von Echtzeit-Sensordaten;
Bestimmen (908), basierend auf der Priorität, einer Position der zweiten Echtzeit-Sensordaten auf der GUI;
Anzeigen (910), auf der GUI des externen Defibrillators, eines zweiten Vitalzeichenkanals, wobei der zweite Vitalzeichenkanal die zweite Eingabe von Echtzeit-Sensordaten umfasst, wobei sich der zweite Vitalzeichenkanal an der bestimmten Position auf der GUI befindet und wobei der zweite Vitalzeichenkanal dazu konfiguriert ist, Positionen auf der GUI zu ändern;
Erfassen (912) einer Benutzereingabe, wobei die Benutzereingabe eine Auswahl entweder aus dem ersten Vitalzeichenkanal oder dem zweiten Vitalzeichenkanal ist;
Ändern (914), auf der GUI, mindestens eines Merkmals des ausgewählten Vitalzeichenkanals, wobei das mindestens eine Merkmal eine Position des ausgewählten Vitalzeichenkanals, eine Breite des ausgewählten Vitalzeichenkanals und eine Bezeichnung des ausgewählten Vitalzeichenkanals umfasst;
Anzeigen (916), auf der GUI des externen Defibrillators, des ersten Vitalzeichenkanals und des zweiten Vitalzeichenkanals mit dem geänderten Merkmal des ausgewählten Vitalzeichenkanals;
Erfassen, dass der zweite Sensor von dem externen Defibrillator entfernt wurde;
Anzeigen einer Warnung, dass der zweite Sensor von dem externen Defibrillator entfernt wurde;
Erfassen, dass eine Entfernungsoption über die GUI für den zweiten Vitalzeichenkanal ausgewählt wurde;
als Reaktion auf das Erfassen, dass die Entfernungsoption für den zweiten Vitalzeichenkanal ausgewählt wurde, Anzeigen einer Dialogbestätigung zum Entfernen des zweiten Vitalzeichenkanals;
Erfassen, dass die Dialogbestätigung über die GUI ausgewählt wurde; und
Entfernen des zweiten Vitalzeichenkanals von der GUI.

2. Verfahren (900) nach Anspruch 1, das weiter Folgendes umfasst:
Bestimmen, dass die Priorität der zweiten Eingabe von Echtzeit-Sensordaten höher ist als die einer Priorität der ersten Eingabe von Echtzeit-Sensordaten;
Anzeigen des zweiten Vitalzeichenkanals an einer Position oberhalb des ersten Vitalzeichenkanals; und
Anzeigen des ersten Vitalzeichenkanals in einer zweiten Position auf der GUI.

3. Verfahren (900) nach Anspruch 1 oder 2, das weiter Folgendes umfasst:
Erfassen, dass ein dritter Sensor mit dem externen Defibrillator verbunden ist, wobei der dritte Sensor eine dritte Eingabe von Echtzeit-Sensordaten sammelt;
Bestimmen einer Priorität der dritten Eingabe von Echtzeit-Sensordaten im Vergleich zu der ersten Eingabe von Echtzeit-Sensordaten und der zweiten Eingabe von Echtzeit-Sensordaten;
Bestimmen, basierend auf der Priorität, einer Position der dritten Echtzeit-Sensordaten auf der GUI;
Bestimmen neu priorisierter Positionen für die ersten Echtzeit-Sensordaten und die zweiten Echtzeit-Sensordaten;
Anzeigen, auf der GUI des externen Defibrillators, eines dritten Vitalzeichenkanals, wobei der dritte Vitalzeichenkanal die dritte Eingabe von Echtzeit-Sensordaten umfasst, wobei sich der dritte Vitalzeichenkanal an der bestimmten Position auf der GUI befindet, und wobei der dritte Vitalzeichenkanal dazu konfiguriert ist, Positionen auf der GUI zu ändern; und
Anzeigen des ersten Vitalzeichenkanals und des zweiten Vitalzeichenkanals in den neu priorisierten Positionen der GUI.

4. Verfahren (900) nach einem der Ansprüche 1 bis 3, wobei die erste Eingabe von Echtzeit-Sensordaten auf dem ersten Vitalzeichenkanal eine EKG-Wellenform ist, wobei der erste Vitalzeichenkanal ständig auf der GUI angezeigt wird, und wobei der erste Vitalzeichenkanal an die erste Position an der Oberseite der GUI angeheftet ist.

5. Verfahren (900) nach einem der Ansprüche 1 bis 4, wobei das Erfassen der Benutzereingabe ferner das Erfassen einer Berührungs-Zieh- und-Ablegebewegung über die GUI umfasst, und wobei die Berührungs-Zieh-und-Ablegebewegung das Ändern der Position des ausgewählten Vitalzeichenkanals umfasst.

6. Verfahren (900) nach einem der Ansprüche 1 bis 5, das ferner das Anzeigen auf der GUI eines Menüs umfasst, wobei das Menü auswählbare Optionen für eine Patientenbehandlungsereignisliste und Hinzufügen eines Ereignisses umfasst.

7. Verfahren (900) nach Anspruch 6, das weiter Folgendes umfasst:
a) Überwachen von Vitalzeichen über den mindestens einen Sensor, der an den externen Defibrillator gekoppelt ist;
Bestimmen eines Vitalzeichenereignisses basierend auf Daten von dem mindestens einen Sensor, wobei das Vitalzeichenereignis mindestens eines von einem Schock, einer Stimulationseinstellung, einer beendeten Stimulation, einer gestarteten Stimulation, einer geänderten Stimulation, einer pausierten Stimulation, einer Alarmauslösung, einem Drucken und einer 12-/15-Kanal-Ableitung umfasst;
Hinzufügen des Vitalzeichenereignisses zu der Patientenbehandlungsereignisliste;
Erfassen einer Auswahl der Patientenbehandlungsereignisliste aus einem Menü;
und Anzeigen auf der GUI des externen Defibrillators, der Patientenbehandlungsereignisliste, wobei die Patientenbehandlungsereignisliste Patienteninformationen, eine Vitalzeichenereignisliste und einen Link zu einer Wellenform umfasst, die dem Vitalzeichenereignis zugeordnet ist; und/oder
b) Erfassen einer Benutzeranforderung zum Hinzufügen eines Ereignisses zu der Patientenbehandlungsereignisliste, wobei das Ereignis mindestens eines einer Arzneimittelabgabe und einer Behandlung umfasst; und/oder
c) Sortieren der Patientenbehandlungsereignisliste basierend auf dem Zeitpunkt, zu dem das Ereignis aufgetreten ist, wobei das Sortieren entweder in aufsteigender oder absteigender Folge von dem Anfang einer aktuellen Patientenepisode verläuft; und/oder
d) Filtern der Patientenbehandlungsereignisliste basierend auf mindestens einem von Behandlungen, Arzneimitteln, generischen Ereignissen und 12-/15-Kanal-Ableitung.

8. Externer Defibrillator (102, 202, 302), der Folgendes umfasst:
mindestens einen Sensor (118), der abnehmbar an den externen Defibrillator gekoppelt ist;
eine GUI (402), die dazu konfiguriert ist, eine Vielzahl von Patientendaten anzuzeigen, wobei die GUI Folgendes umfasst:
einen ersten Vitalzeichenkanal (420), wobei der erste Vitalzeichenkanal eine erste Eingabe von Echtzeit-Sensordaten (464) umfasst, die von dem mindestens einen Sensor gesammelt werden, und wobei sich der erste Vitalzeichenkanal an einer ersten Position (454) auf der GUI befindet;
einen zweiten Vitalzeichenkanal (450), wobei der zweite Vitalzeichenkanal eine zweite Eingabe von Echtzeit-Sensordaten (468) umfasst, die von dem mindestens einen Sensor gesammelt werden, wobei sich der zweite Vitalzeichenkanal an einer zweiten Position (460) auf der GUI befindet, und wobei der zweite Vitalzeichenkanal dazu konfiguriert ist, die Positionen zu ändern;
einen dritten Vitalzeichenkanal (452), wobei der dritte Vitalzeichenkanal eine Eingabe von Echtzeit-Sensordaten (470) umfasst, die von dem mindestens einen Sensor gesammelt werden, und wobei sich der dritte Vitalzeichenkanal an einer dritten Position (462) auf der GUI befindet, und wobei der dritte Vitalzeichenkanal dazu konfiguriert ist, die Positionen zu ändern; und
ein Menü, wobei das Menü eine Vielzahl auswählbarer Optionen umfasst, die eine Patientenbehandlungsereignisliste beinhalten, und wobei die Patientenbehandlungsereignisliste mindestens einen Verweis auf ein Ereignis in der ersten Eingabe von Echtzeit-Sensordaten beinhaltet;
**dadurch gekennzeichnet, dass** der externe Defibrillator ferner einen Prozessor umfasst, der dazu konfiguriert ist, das Verfahren nach einem der vorstehenden Ansprüche durchzuführen.

9. Externer Defibrillator nach Anspruch 8, wobei die erste Eingabe von Echtzeit-Sensordaten (464) eine EKG-Wellenform ist.

10. Externer Defibrillator nach Anspruch 8 oder 9, wobei der erste Vitalzeichenkanal (420) an der ersten Position (454) angeheftet ist, der zweite Vitalzeichenkanal (450) an der dritten Position (460) ist, und der dritte Vitalzeichenkanal (452) an der zweiten Position (462) ist.

11. Externer Defibrillator nach Anspruch 8, 9 oder 10, wobei die erste Eingabe von Echtzeit-Sensordaten (464) mindestens eines von einer EKG-Wellenform, einer EtCO2-Wellenform, einer SpO2-Wellenform, einer Temperatur, einer Herzfrequenz, einer Carboxyhämoglobinkonzentration, einer Methämoglobinkonzentration, einer Atemfrequenz, CO2, einem invasiven Blutdruck und einem nichtinvasiven Blutdruck ist, wobei die zweite Eingabe von Echtzeit-Sensordaten (468) mindestens eines von einer EKG-Wellenform, einer EtCO2-Wellenform, einer SpO2-Wellenform, einer Temperatur, einem invasiven Blutdruck und einem nichtinvasiven Blutdruck ist, und wobei die dritte Eingabe von Echtzeit-Sensordaten (470) mindestens eines von einer EKG-Wellenform, einer EtCO2-Wellenform, einer SpO2-Wellenform, einer Temperatur, einem invasiven Blutdruck und einem nichtinvasiven Blutdruck ist, wobei beispielsweise die erste Eingabe von Echtzeit-Sensordaten (464) eine EKG-Wellenform ist, die zweite Eingabe von Echtzeit-Sensordaten (468) eine EKG-Wellenform ist und die dritte Eingabe von Echtzeit-Sensordaten (470) eine EKG-Wellenform ist, und wobei einer von dem ersten Vitalzeichenkanal (420), dem zweiten Vitalzeichenkanal (450) oder dem dritten Vitalzeichenkanal (452) an der ersten Position (454) oben in der GUI angeordnet ist.

12. Externer Defibrillator nach einem der Ansprüche 8 bis 11, wobei der erste Vitalzeichenkanal (420), der zweite Vitalzeichenkanal (450) und der dritte Vitalzeichenkanal (452) jeweils eine Breite umfassen, und wobei die Breite mindestens eine von einer vollen Breite, einer halben Breite und einer Viertelbreite ist,
wobei bei voller Breite optional der erste Vitalzeichenkanal (420) der zweite Vitalzeichenkanal (450) und der dritte Vitalzeichenkanal (452) einen Trend beinhalten, und bei halber Breite oder Viertelbreite der erste Vitalzeichenkanal (420), der zweite Vitalzeichenkanal (450) und der dritte Vitalzeichenkanal (452) den Trend nicht beinhalten; und/oder
wobei die Patientenbehandlungsereignisliste ferner Patienteninformationen, eine Ereignisliste und einen Link zu einer mit dem Ereignis verbundenen Wellenform umfasst, und wobei die Ereignisliste nach Zeitreihenfolge sortiert ist.

13. Nichtflüchtiges computerlesbares Speichermedium, das computerausführbare Anweisungen, die darauf gespeichert sind, aufweist, die, wenn sie von einem oder mehreren Prozessoren eines externen Defibrillators ausgeführt werden, die Prozessoren veranlassen, Operationen durchzuführen, die Folgendes umfassen:
Anzeigen auf einer GUI des externen Defibrillators, eines ersten Vitalzeichenkanals, wobei der erste Vitalzeichenkanal eine erste Eingabe von Echtzeit-Sensordaten umfasst, die von mindestens einem Sensor gesammelt werden, und wobei sich der erste Vitalzeichenkanal an einer ersten Position auf der GUI befindet;
Erfassen, dass ein zweiter Sensor mit dem externen Defibrillator verbunden ist, wobei der zweite Sensor eine zweite Eingabe von Echtzeit-Sensordaten sammelt;
Bestimmen einer Priorität der zweiten Eingabe von Echtzeit-Sensordaten im Vergleich zu der ersten Eingabe von Echtzeit-Sensordaten;
Bestimmen, basierend auf der Priorität, einer Position der zweiten Echtzeit-Sensordaten auf der GUI;
Anzeigen, auf der GUI des externen Defibrillators, eines zweiten Vitalzeichenkanals, wobei der zweite Vitalzeichenkanal die zweite Eingabe von Echtzeit-Sensordaten umfasst, wobei sich der zweite Vitalzeichenkanal an der bestimmten Position auf der GUI befindet und wobei der zweite Vitalzeichenkanal dazu konfiguriert ist, Positionen auf der GUI zu ändern;
Erfassen einer Benutzereingabe, wobei die Benutzereingabe eine Auswahl entweder aus dem ersten Vitalzeichenkanal oder dem zweiten Vitalzeichenkanal ist;
Ändern, auf der GUI, mindestens eines Merkmals des ausgewählten Vitalzeichenkanals, wobei das mindestens eine Merkmal eine Position des ausgewählten Vitalzeichenkanals, eine Breite des ausgewählten Vitalzeichenkanals und eine Bezeichnung des ausgewählten Vitalzeichenkanals umfasst;
Anzeigen, auf der GUI des externen Defibrillators, des ersten Vitalzeichenkanals und des zweiten Vitalzeichenkanals mit dem geänderten Merkmal des ausgewählten Vitalzeichenkanals;
Erfassen, dass der zweite Sensor von dem externen Defibrillator entfernt wurde;
Anzeigen einer Warnung, dass der zweite Sensor von dem externen Defibrillator entfernt wurde;
Erfassen, dass eine Entfernungsoption über die GUI für den zweiten Vitalzeichenkanal ausgewählt wurde;
als Reaktion auf das Erfassen, dass die Entfernungsoption für den zweiten Vitalzeichenkanal ausgewählt wurde, Anzeigen einer Dialogbestätigung zum Entfernen des zweiten Vitalzeichenkanals;
Erfassen, dass die Dialogbestätigung über die GUI ausgewählt wurde; und
Entfernen des zweiten Vitalzeichenkanals von der GUI.

14. Nichtflüchtiges computerlesbares Medium nach Anspruch 13, wobei die Operationen ferner das automatische Einsetzen des Anzeigens des ersten Vitalzeichenkanals, des zweiten Vitalzeichenkanals, eines dritten Vitalzeichenkanals eines vierten Vitalzeichenkanals, eines fünften Vitalzeichenkanals und eines sechsten Vitalzeichenkanals auf der GUI umfassen; und/oder
wobei die erste Eingabe von Echtzeit-Sensordaten auf dem ersten Vitalzeichenkanal eine EKG-Wellenform ist, wobei der erste Vitalzeichenkanal ständig auf der GUI angezeigt wird, und wobei der erste Vitalzeichenkanal an die erste Position an der Oberseite der GUI angeheftet ist.

## Revendications

1. Procédé (900) comprenant :
l'affichage (902), sur une interface utilisateur graphique (GUI) d'un défibrillateur externe, d'un premier canal de signes vitaux, ledit premier canal de signes vitaux comprenant une première entrée de données de capteur en temps réel recueillies par au moins un capteur, et ledit premier canal de signes vitaux étant dans une première position sur la GUI ;
la détection (904) qu'un deuxième capteur est connecté au défibrillateur externe, ledit deuxième capteur recueillant une deuxième entrée de données de capteur en temps réel ;
la détermination (906) d'une priorité de la deuxième entrée de données de capteur en temps réel par rapport à la première entrée de données de capteur en temps réel ;
la détermination (908), sur la base de la priorité, d'une position des deuxièmes données de capteur en temps réel sur la GUI,
l'affichage (910), sur la GUI du défibrillateur externe, d'un deuxième canal de signes vitaux, ledit deuxième canal de signes vitaux comprenant la deuxième entrée de données de capteur en temps réel, ledit deuxième canal de signes vitaux étant dans la position déterminée sur la GUI, et ledit deuxième canal de signes vitaux étant configuré pour changer de position sur la GUI ;
la détection (912) d'une entrée utilisateur, ladite entrée utilisateur étant une sélection soit du premier canal de signes vitaux, soit du deuxième canal de signes vitaux ;
la modification (914) sur la GUI d'au moins une caractéristique du canal de signes vitaux sélectionné, ladite au moins une caractéristique comprenant une position du canal de signes vitaux sélectionné, une largeur du canal de signes vitaux sélectionné, et une désignation du canal de signes vitaux sélectionné ;
l'affichage (916), sur la GUI du défibrillateur externe, du premier canal de signes vitaux et du deuxième canal de signes vitaux avec la caractéristique modifiée du canal de signes vitaux sélectionné ;
la détection que le deuxième capteur a été retiré du défibrillateur externe ;
l'affichage d'une alerte indiquant que le deuxième capteur a été retiré du défibrillateur externe ;
la détection qu'une option de suppression a été sélectionnée par le biais de la GUI pour le deuxième canal de signes vitaux ;
en réponse à la détection que l'option de suppression a été sélectionnée pour le deuxième canal de signes vitaux, l'affichage d'une boîte de dialogue de confirmation pour supprimer le deuxième canal de signes vitaux ;
la détection que la confirmation de la boîte de dialogue a été sélectionnée par le biais de la GUI ;
et la suppression du deuxième canal de signes vitaux de la GUI.

2. Procédé (900) selon la revendication 1, comprenant en outre :
la détermination que la priorité de la deuxième entrée de données de capteur en temps réel est supérieure à une priorité de la première entrée de données de capteur en temps réel ;
l'affichage du deuxième canal de signes vitaux dans une position au-dessus du premier canal de signes vitaux ; et
l'affichage du premier canal de signes vitaux dans une deuxième position sur la GUI.

3. Procédé (900) selon la revendication 1 ou la revendication 2, comprenant en outre :
la détection qu'un troisième capteur est connecté au défibrillateur externe, ledit troisième capteur recueillant une troisième entrée de données de capteur en temps réel ;
la détermination d'une priorité de la troisième entrée de données de capteur en temps réel par rapport à la première entrée de données de capteur en temps réel et à la deuxième entrée de données de capteur en temps réel ;
la détermination, sur la base de la priorité, d'une position des troisièmes données de capteur en temps réel sur la GUI,
la détermination de positions repriorisées pour la première entrée de données de capteur en temps réel et la deuxième entrée de données de capteur en temps réel ;
l'affichage, sur la GUI du défibrillateur externe, d'un troisième canal de signes vitaux, ledit troisième canal de signes vitaux comprenant la troisième entrée de données de capteur en temps réel, ledit troisième canal de signes vitaux étant dans la position déterminée sur la GUI, et ledit troisième canal de signes vitaux étant configuré pour changer de position sur la GUI ; et
l'affichage du premier canal de signes vitaux et du deuxième canal de signes vitaux dans les positions repriorisées sur la GUI.

4. Procédé (900) selon l'une quelconque des revendications 1 à 3, dans lequel la première entrée de données de capteur en temps réel sur le premier canal de signes vitaux est une courbe ECG, ledit premier canal de signes vitaux étant affiché en permanence sur la GUI, et ledit premier canal de signes vitaux étant épinglé à la première position en haut de la GUI.

5. Procédé (900) selon l'une quelconque des revendications 1 à 4, dans lequel la détection de l'entrée utilisateur comprend en outre la détection d'un mouvement tactile de glisser-déposer sur la GUI, et dans lequel ledit mouvement tactile de glisser-déposer comprend la modification de la position du canal de signes vitaux sélectionné.

6. Procédé (900) selon l'une quelconque des revendications 1 à 5, comprenant en outre l'affichage d'un menu sur la GUI, ledit menu comprenant des options sélectionnables pour une liste d'événements de soins au patient et l'ajout d'un événement.

7. Procédé (900) selon la revendication 6, comprenant en outre :
a) la surveillance des signes vitaux par le biais dudit au moins un capteur couplé au défibrillateur externe ;
la détermination, sur la base de données provenant dudit au moins un capteur, d'un événement de signe vital, ledit événement de signe vital comprenant au moins l'un parmi un choc, un réglage de stimulation, stimulation arrêtée, stimulation démarrée, stimulation modifiée, stimulation en pause, violation d'alarme, impression et ECG 12/15 dérivations ;
l'ajout de l'événement de signe vital à la liste d'événements de soins au patient ;
la détection d'une sélection de la liste d'événements de soins au patient à partir d'un menu ; et
l'affichage, sur la GUI du défibrillateur externe, de la liste d'événements de soins au patient, ladite liste d'événements de soins au patient comprenant des informations patient, une liste d'événements de signes vitaux, et un lien vers une courbe associée à l'événement de signe vital ; et/ou
b) la détection d'une demande utilisateur d'ajouter un événement à la liste d'événements de soins au patient, ledit événement comprenant au moins l'un parmi une administration de médicament et un traitement ; et/ou
c) le tri de la liste d'événements de soins au patient sur la base de l'heure à laquelle l'événement s'est produit, ledit tri se faisant soit par ordre croissant, soit par ordre décroissant depuis le début d'un épisode patient en cours ; et/ou
d) le filtrage de la liste d'événements de soins au patient sur la base d'au moins l'un parmi des traitements, des médicaments, des événements génériques et des ECG 12/15 dérivations.

8. Défibrillateur externe (102, 202, 302) comprenant :
au moins un capteur (118) couplé de manière amovible au défibrillateur externe ;
une interface utilisateur graphique (GUI) (402) configurée pour afficher une pluralité de données patient, l'interface utilisateur graphique comprenant :
un premier canal de signes vitaux (420), ledit premier canal de signes vitaux comprenant une première entrée de données de capteur en temps réel (464) recueillies par ledit au moins un capteur, et ledit premier canal de signes vitaux étant dans une première position (454) sur l'interface utilisateur graphique ;
un deuxième canal de signes vitaux (450), ledit deuxième canal de signes vitaux comprenant une deuxième entrée de données de capteur en temps réel (468) recueillies par ledit au moins un capteur, ledit deuxième canal de signes vitaux étant dans une deuxième position (460) sur l'interface utilisateur graphique, et ledit deuxième canal de signes vitaux étant configuré pour changer de position ;
un troisième canal de signes vitaux (452), ledit troisième canal de signes vitaux comprenant une troisième entrée de données de capteur en temps réel (470) recueillies par ledit au moins un capteur, ledit troisième canal de signes vitaux étant dans une troisième position (462) sur l'interface utilisateur graphique, et ledit troisième canal de signes vitaux étant configuré pour changer de position ; et
un menu, ledit menu comprenant une pluralité d'options sélectionnables incluant une liste d'événements de soins au patient, et ladite liste d'événements de soins au patient comprenant au moins une référence à un événement dans la première entrée de données de capteur en temps réel ;
**caractérisé en ce que** le défibrillateur externe comprend en outre un processeur configuré pour exécuter le procédé selon l'une quelconque des revendications précédentes.

9. Défibrillateur externe selon la revendication 8, dans lequel la première entrée de données de capteur en temps réel (464) est une courbe ECG.

10. Défibrillateur externe selon la revendication 8 ou la revendication 9, dans lequel le premier canal de signes vitaux (420) est épinglé à la première position (454), le deuxième canal de signes vitaux (450) est dans la troisième position (460), et le troisième canal de signes vitaux (452) est dans la deuxième position (462).

11. Défibrillateur externe selon la revendication 8, 9 ou 10, dans lequel la première entrée de données de capteur en temps réel (464) est au moins l'une parmi une courbe ECG, une courbe EtCO2, une courbe SpO2, la température, la fréquence cardiaque, la concentration en carboxyhémoglobine, la concentration en méthémoglobine, la fréquence respiratoire, le CO2, la pression artérielle invasive et la pression artérielle non invasive, dans lequel la deuxième entrée de données de capteur en temps réel (468) est au moins l'une parmi une courbe ECG, une courbe EtCO2, une courbe SpO2, la température, la pression artérielle invasive et la pression artérielle non invasive, et dans lequel la troisième entrée de données de capteur en temps réel (470) est au moins l'une parmi une courbe ECG, une courbe EtCO2, une courbe SpO2, la température, la pression artérielle invasive et la pression artérielle non invasive, par exemple dans lequel la première entrée de données de capteur en temps réel (464) est une courbe ECG, la deuxième entrée de données de capteur en temps réel (468) est une courbe ECG et la troisième entrée de données de capteur en temps réel (470) est une courbe ECG, et dans lequel l'un parmi le premier canal de signes vitaux (420), le deuxième canal de signes vitaux (450) ou le troisième canal de signes vitaux (452) est dans la première position (454) en haut de l'interface utilisateur graphique.

12. Défibrillateur externe selon l'une quelconque des revendications 8 à 11, dans lequel chacun du premier canal de signes vitaux (420), du deuxième canal de signes vitaux (450) et du troisième canal de signes vitaux (452) comprend une largeur, et dans lequel la largeur est au moins l'une parmi la largeur totale, la demi-largeur et le quart de largeur,
dans lequel, éventuellement, en largeur totale, le premier canal de signes vitaux (420), le deuxième canal de signes vitaux (450) et le troisième canal de signes vitaux (452) comprennent une tendance, et en demi-largeur ou quart de largeur, le premier canal de signes vitaux (420), le deuxième canal de signes vitaux (450) et le troisième canal de signes vitaux (452) ne comprennent pas la tendance ; et/ou
dans lequel la liste d'événements de soins au patient comprend en outre des informations patient, une liste d'événements et un lien vers une courbe associée à l'événement, et dans lequel la liste d'événements est triée par ordre chronologique.

13. Support non transitoire lisible par ordinateur comportant des instructions exécutables par ordinateur qui, si elles sont exécutées par un ou plusieurs processeurs d'un défibrillateur externe, amènent les processeurs à effectuer des opérations, comprenant :
afficher, sur une interface graphique (GUI) du défibrillateur externe, un premier canal de signes vitaux, ledit premier canal de signes vitaux comprenant une première entrée de données de capteur en temps réel recueillies par au moins un capteur, et ledit premier canal de signes vitaux étant dans une première position sur la GUI ;
détecter qu'un deuxième capteur est connecté au défibrillateur externe, ledit deuxième capteur recueillant une deuxième entrée de données de capteur en temps réel ;
déterminer une priorité de la deuxième entrée de données de capteur en temps réel par rapport à la première entrée de données de capteur en temps réel ;
déterminer, sur la base de la priorité, une position des deuxièmes données de capteur en temps réel sur la GUI,
afficher, sur la GUI du défibrillateur externe, un deuxième canal de signes vitaux, ledit deuxième canal de signes vitaux comprenant la deuxième entrée de données de capteur en temps réel, ledit deuxième canal de signes vitaux étant dans la position déterminée sur la GUI, et ledit deuxième canal de signes vitaux étant configuré pour changer de position sur la GUI,
détecter une entrée utilisateur, ladite entrée utilisateur étant une sélection soit du premier canal de signes vitaux, soit du deuxième canal de signes vitaux ;
modifier sur la GUI au moins une caractéristique du canal de signes vitaux sélectionné, ladite au moins une caractéristique comprenant une position du canal de signes vitaux sélectionné, une largeur du canal de signes vitaux sélectionné, et une désignation du canal de signes vitaux sélectionné ;
afficher, sur la GUI du défibrillateur externe, le premier canal de signes vitaux et le deuxième canal de signes vitaux avec la caractéristique modifiée du canal de signes vitaux sélectionné ;
détecter que le deuxième capteur a été retiré du défibrillateur externe ;
afficher une alerte indiquant que le deuxième capteur a été retiré du défibrillateur externe ;
détecter qu'une option de suppression a été sélectionnée par le biais de la GUI pour le deuxième canal de signes vitaux ;
en réponse à la détection que l'option de suppression a été sélectionnée pour le deuxième canal de signes vitaux, afficher une confirmation de dialogue pour supprimer le deuxième canal de signes vitaux ;
détecter que la confirmation de dialogue a été sélectionnée par le biais de la GUI ; et
supprimer le deuxième canal de signes vitaux de la GUI.

14. Support non transitoire lisible par ordinateur selon la revendication 13, dans lequel les opérations comprennent en outre l'utilisation automatique de l'affichage du premier canal de signes vitaux, du deuxième canal de signes vitaux, d'un troisième canal de signes vitaux, d'un quatrième canal de signes vitaux, d'un cinquième canal de signes vitaux et d'un sixième canal de signes vitaux sur la GUI ; et/ou
dans lequel la première entrée de données de capteur en temps réel sur le premier canal de signes vitaux est une courbe ECG, ledit premier canal de signes vitaux étant affiché en permanence sur la GUI, et ledit premier canal de signes vitaux étant épinglé à la première position en haut de la GUI.
